(19) Europäisches Patentamt
European Patent Office
Office européen des brevets

(11) EP 4 691 387 A1

(12) EUROPEAN PATENT APPLICATION

(43) Date of publication:
11.02.2026 Bulletin 2026/07

(51) International Patent Classification (IPC):
A61B 17/32 $^{(2006.01)}$   A61B 34/20 $^{(2016.01)}$
A61B 34/30 $^{(2016.01)}$   A61B 17/00 $^{(2006.01)}$
A61B 90/00 $^{(2016.01)}$

(21) Application number: 25193732.2

(22) Date of filing: 04.08.2025

(52) Cooperative Patent Classification (CPC):
A61B 17/320068; A61B 34/20; A61B 34/30;
A61B 34/32; A61B 34/35; A61B 34/76;
A61B 2017/00022; A61B 2034/105;
A61B 2034/2051; A61B 2034/2055;
A61B 2034/2059; A61B 2034/2063; A61B 2090/064;
A61B 2090/065

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 05.08.2024 US 202463679295 P

(71) Applicant: Stryker Corporation
Portage, MI 49002 (US)

(72) Inventors:
• WEEDE, Oliver
 79110 Freiburg (DE)
• PFEIL, Antoine
 67530 Klingenthal (FR)
• SCHETTINO, Vincenzo
 86150 Augsburg (DE)
• DOWNEY, Adam
 Kalamazoo, 49009 (US)

(74) Representative: Schott, Jakob Valentin
Wuesthoff & Wuesthoff
Patentanwälte und Rechtsanwalt PartG mbB
Schweigerstraße 2
81541 München (DE)

(54) ROBOTICALLY CONTROLLED ULTRASONIC INSTRUMENT

(57) Systems and methods for operating a robotic manipulator coupled to an ultrasonic instrument to control movement of the ultrasonic instrument to resect a target volume of patient tissue. The ultrasonic instrument is driven with an AC drive signal that induces vibrations in a tip of the ultrasonic instrument, and is positioned so that a distal region of the tip is vibrating against a portion of the patient tissue. A characteristic of the AC drive signal is obtained when the distal region of the tip is vibrating against the tissue portion, and a determination is made of whether the tissue portion corresponds to the target volume based on the determined characteristic. The robotic manipulator is operated to control movement of the ultrasonic instrument relative to the patient tissue based on the determination.

FIG. 1

EP 4 691 387 A1

**Description**

CROSS-REFERENCE TO RELATED APPLICATIONS

The subject application claims priority to and all benefits of U.S. Provisional Patent App. No. 63/679,295, filed August 5, 2024, the entire contents of which are hereby incorporated by reference.

BACKGROUND

In the medical field, there are numerous procedures that involve the removal or alteration of structures within the body to alleviate pain or correct imbalances in the spine. These procedures include, but are not limited to, discectomies, bone spur resections, foraminotomies, laminectomies, pedicle subtraction osteotomies, facetectomies, and pre-drilling of screw trajectories for pedicle screws. Traditionally, these procedures have been performed using a freehand technique, where the surgeon manually removes or alters the structures using tools like burs, saws, and drills, and relies on visual feedback and feel to judge whether all targeted or problematic material has been removed. Other procedures, such as pedicle screw and cage insertions, are also frequently performed using manual tools that rely on the surgeon's interpretation of visual feedback and feel.

Freehand techniques can be supplemented with surgical navigation, which generally tracks the position of a surgical tool relative to patient anatomy for visualization on a display in the surgeon's view. This technique provides the surgeon with more precise information about the location of the structures to be removed or altered, but still relies heavily on the surgeon's skill and judgement. In some surgical environments, the surgical navigation information may also be used to control a robotic manipulator coupled to the tool, such as to guide and/or limit movement of the tool by the surgeon, or perform the procedure autonomously with limited surgeon interaction.

Despite the advancements in surgical techniques provided by surgical navigation and robotic surgery, there remains a need for more precise, reliable, and less invasive methods for performing these procedures.

SUMMARY

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes a method for operating a robotic manipulator coupled to an ultrasonic instrument to control movement of the ultrasonic instrument to resect a target volume of patient tissue, the method including driving the ultrasonic instrument with an AC drive signal to induce vibrations in a tip of the ultrasonic instrument, and positioning the ultrasonic instrument so that a distal region of the tip is vibrating against a portion of the patient tissue. The method also includes obtaining a first characteristic of the AC drive signal when the distal region of the tip is vibrating against the tissue portion, and determining whether the tissue portion corresponds to the target volume based on the first characteristic. The method also includes operating the robotic manipulator to control movement of the ultrasonic instrument relative to the patient tissue based on the determination of whether the tissue portion corresponds to the target volume. Other implementations of this aspect include corresponding computer systems, apparatus, and computer programs recorded on one or more computer storage devices, each configured to perform the actions of the methods.

Another general aspect includes a method for operating a robotic manipulator coupled to an ultrasonic instrument to control movement of the ultrasonic instrument to resect a target volume of patient tissue, the method including receiving a medical image of the patient tissue and determining a tissue-density map for the patient tissue based on the medical image. The method also includes generating tracking data indicative of a position of a distal region of a tip of the ultrasonic instrument relative to the patient tissue with a surgical navigation system, and determining that the tracking data indicates the distal region of the tip is contacting a portion of the target volume. The method also includes, responsive to determining that the tracking data indicates the distal region of the tip is contacting a portion of the target volume: determining a target force to apply to the ultrasonic instrument for resection of the target volume portion based on a portion of the tissue-density map corresponding to the target volume portion, driving the ultrasonic instrument with an AC drive signal to induce vibrations in the tip of the ultrasonic instrument for resecting the target volume portion, obtaining a characteristic of the AC drive signal, determining a measure of force being applied to the ultrasonic instrument based on the tissue-density map portion and the characteristic, and operating the robotic manipulator to regulate the force applied to the ultrasonic instrument when the tracking data indicates the distal region of the tip is vibrating against the target volume portion based on the target force and the determined force measure. Other implementations of this aspect include corresponding computer systems, apparatus, and computer programs recorded on one or more computer storage devices, each configured to perform the actions of the methods.

Another general aspect includes a method for operating a robotic manipulator coupled to an ultrasonic instrument to control movement of the ultrasonic instrument to resect patient tissue, the method including receiving a medical image of patient tissue including tissue targeted for resection and tissue to be avoided, and generating a virtual map in a known coordinate system based on the medical image. The virtual map defines: a plurality of first regions of the known coordinate system that correspond to the targeted tissue according to the medical image, each of the first regions being associated with a first control property initially set to attract a distal region of a tip of the ultrasonic instrument towards the first region, and a plurality of second regions of the known coordinate system that correspond to the tissue to be avoided according to the medical image, each of the second regions being associated with a second control property initially set to resist movement of the distal region of the tip towards the second region. The method also includes driving the ultrasonic instrument with an AC drive signal to induce vibrations in the tip of the ultrasonic instrument, generating tracking data indicative of a position of the distal region of the tip in the known coordinate system with a surgical navigation system, and operating the robotic manipulator to control movement of the distal region of the tip to a first of the first regions based on the tracking data and the virtual map. The method also includes obtaining a first characteristic of the AC drive signal when the tracking data indicates the distal region of the tip is vibrating in the first of the first regions, and determining that the first characteristic indicates contact by the distal region of the tip with the targeted tissue. The method also includes, responsive to determining that the first characteristic indicates contact by the distal region of the tip with the targeted tissue: operating the ultrasonic instrument to resect the targeted tissue from the first of the first regions, and setting the first control property associated with the one of the first regions to not attract or resist movement of the distal region of the tip towards the first of the first regions. Other implementations of this aspect include corresponding computer systems, apparatus, and computer programs recorded on one or more computer storage devices, each configured to perform the actions of the methods.

Another general aspect includes a robotic surgical system including an ultrasonic instrument having an irrigation pathway, a tip, and an ultrasonic transducer coupled to the tip, the ultrasonic transducer configured to vibrate the tip responsive to receiving an AC drive signal. The system also includes a power supply coupled to the ultrasonic instrument and configured to generate the AC drive signal applied to the ultrasonic transducer of the ultrasonic instrument, a robotic manipulator coupled to the ultrasonic instrument and configured to control movement of the ultrasonic instrument to resect a target volume of patient tissue, and a control system coupled the power supply and the robotic manipulator. The control system is configured to: operate the power supply to source the AC drive signal to the ultrasonic instrument to induce vibrations in the tip of the ultrasonic instrument; obtain a first characteristic of the sourced AC drive signal; determine whether a distal region of the tip is vibrating against a portion of the target volume based on the first characteristic; and operate the robotic manipulator to control movement of the ultrasonic instrument relative to the patient tissue based on the determination of whether the distal region of the tip is vibrating against a portion of the target volume.

Another general aspect includes a robotic surgical system including an ultrasonic instrument having an irrigation pathway, a tip, and an ultrasonic transducer coupled to the tip, the ultrasonic transducer configured to vibrate the tip responsive to receiving an AC drive signal. The system also includes a power supply coupled to the ultrasonic instrument and configured to generate the AC drive signal applied to the ultrasonic transducer of the ultrasonic instrument, a robotic manipulator coupled to the ultrasonic instrument and configured to control movement of the ultrasonic instrument to resect a target volume of patient tissue, a navigation system configured to generate tracking data indicative of a pose of a distal region of the tip relative to the patient tissue, and a control system coupled the power supply and the robotic manipulator. The control system is configured to: receive a medical image of the patient tissue, determine a tissue-density map for the patient tissue based on the medical image, and determine whether the tracking data indicates the distal region of the tip is contacting a portion of the target volume. Responsive to determining that the tracking data indicates the distal region of the tip is contacting a portion of the target volume, the control system is configured to: determine a target force to apply to the ultrasonic instrument for resection of the target volume portion based on a portion of the tissue-density map corresponding to the target volume portion, operate the power supply to source the AC drive signal to the ultrasonic instrument to induce vibrations in the tip of the ultrasonic instrument for resecting the target volume portion, obtain a characteristic of the sourced AC drive signal, determine a measure of force being applied to the ultrasonic instrument based on the tissue-density map portion and the characteristic, and operate the robotic manipulator to regulate the force applied to the ultrasonic instrument based on the target force and the determined force measure.

Another aspect includes a robotic surgical system including an ultrasonic instrument having an irrigation pathway, a tip, and an ultrasonic transducer coupled to the tip, the ultrasonic transducer configured to vibrate the tip responsive to receiving an AC drive signal. The system also includes a power supply coupled to the ultrasonic instrument and configured to generate the AC drive signal applied to the ultrasonic transducer of the ultrasonic instrument, a robotic manipulator coupled to the ultrasonic instrument and configured to guide the ultrasonic instrument to resect a target volume of patient tissue, a navigation system configured to generate tracking data indicative of a position of a distal region of the tip in a known coordinate system, and a control system coupled the power supply and the robotic manipulator. The control system is configured to: receive a medical image of patient tissue including tissue targeted for resection and tissue to be avoided, and generate a virtual map in the known coordinate system based on the medical image. The virtual map defines: a plurality of first regions of the known coordinate system that correspond to the targeted tissue according to the medical image, each of the first regions being associated with a first control property initially set to attract a distal region of a tip of the ultrasonic instrument towards the first region, and a plurality of second regions of the known coordinate system that correspond to the tissue to be avoided according to the medical image, each of the second regions being associated with a second control property initially set to resist movement of the distal region of the tip towards the second region. The control system is also configured to operate the power supply to source the AC drive signal to the ultrasonic instrument to induce vibrations in the tip of the ultrasonic instrument; operate the robotic manipulator to control movement of the distal region of the tip to a first of the first regions based on the tracking data and the virtual map; obtain a first characteristic of the AC drive signal when the tracking data indicates the distal region of the tip is vibrating in the first of the first regions; and determine whether the first characteristic indicates contact by the distal region of the tip with the targeted tissue. The control system is also configured to, responsive to determining that the first characteristic indicates contact by the distal region of the tip with the targeted tissue: operate the ultrasonic instrument to resect the targeted tissue from the first of the first regions, and set the first control property associated with the one of the first regions to not attract or resist movement of the distal region of the tip towards the first of the first regions.

BRIEF DESCRIPTION OF THE DRAWINGS

FIG. 1 illustrates a surgical system including an ultrasonic system with an ultrasonic instrument for resecting and sensing patient tissue, a surgical navigation system for tracking a position of the ultrasonic instrument relative to patient tissue, and a robotic manipulator for maneuvering and/or guiding movement of the ultrasonic instrument relative to the patient tissue.

FIG. 2 illustrates the ultrasonic system in additional detail.

FIG. 3 illustrates the ultrasonic instrument in additional detail.

FIGS. 4A to 4C illustrate distal regions of varying ultrasonic tips that may be coupleable to a handpiece of the ultrasonic instrument.

FIGS. 5A and 5B illustrate electrical circuits modelling current flow through the ultrasonic instrument.

FIG. 6 illustrates components that may be incorporated in the surgical system of FIG. 1.

FIG. 7 illustrates components that may be incorporated into the ultrasonic instrument.

FIGS. 8A and 8B illustrate varying approaches of an ultrasonic instrument relative to patient tissue.

FIGS. 9A and 9B illustrate surgical planning screens of a graphical user interface (GUI) that may be provided by the surgical system of FIG. 1.

FIGS. 10A and 10B illustrates surgical tracking screens of a GUI that may be provided by the surgical system.

DETAILED DESCRIPTION

The present disclosure describes a surgical system and related methods that utilize a robotically controlled ultrasonic instrument for both tissue resection and sensing. Specifically, the ultrasonic instrument may be used to resect patient tissue, aspirate resected patient tissue, irrigate the surgical site, and sense varying types of patient tissue. The robotic manipulation of the ultrasonic instrument may function to improve resection speed and tissue sensing using the ultrasonic instrument, thus enhancing the precision of surgical procedures and reducing the learning curve for effective use of the ultrasonic instrument.

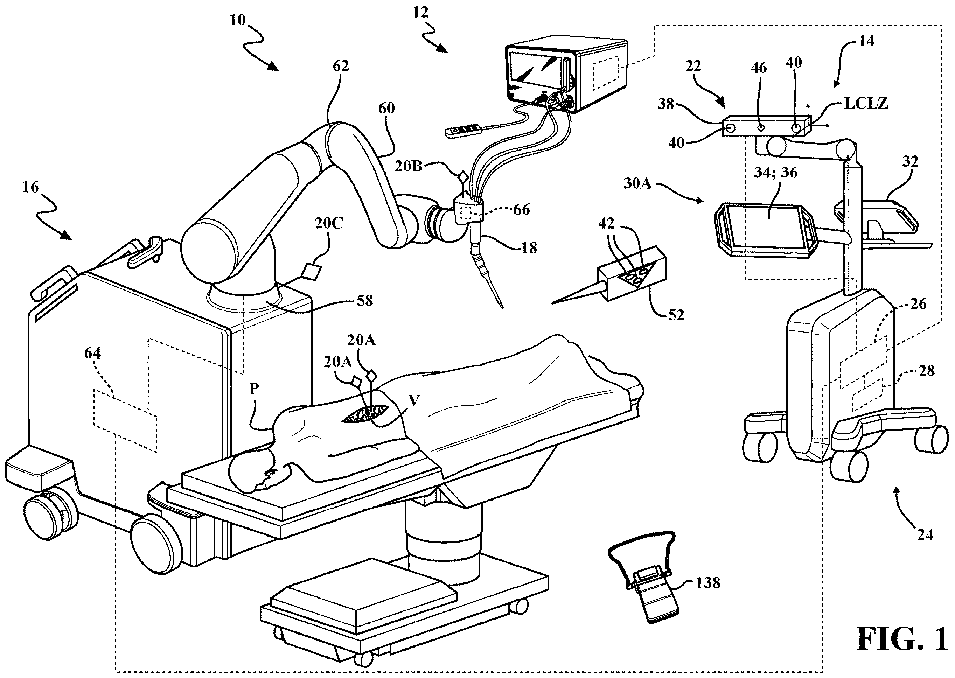

Turning now to the Figures, FIG. 1 illustrates a surgical system 10 for treating a volume of patient tissue targeted for treatment (also referred to herein as a target site). The surgical system 10 may be located in a surgical setting such as an operating room of a medical facility. The surgical system 10 may include an ultrasonic system 12, a surgical navigation system 14, and a robotic manipulator 16.

The robotic manipulator 16 may be coupled to an ultrasonic instrument 18 of the ultrasonic system 12, and may be configured to control movement of the ultrasonic instrument 18 through the surgical workspace to treat a target site. For instance, the robotic manipulator 16, such as at the direction of the surgical navigation system 14, may be configured to maneuver the ultrasonic instrument 18 to treat the target site and/or guide movement of the ultrasonic instrument 18 by a surgeon to treat the target site. In some implementations, the robotic manipulator 16 may be selectively operable in various modes, such as an autonomous mode, a hands-on mode, and a telemanipulation mode.

In the autonomous mode, the surgical navigation system 14 may cause the robotic manipulator 16 to maneuver the ultrasonic instrument 18 to treat the target site while avoiding other objects adjacent the target site, such as other medical tools and adjacent patient tissues including critical structures desired to be avoided (also referred to herein as non-targeted objects), with little or no surgeon interaction. In the hands-on mode, the surgeon may manually control the robotic manipulator 16 by holding and maneuvering an arm of the robotic manipulator 16 to maneuver the ultrasonic instrument 18 to treat the target site, or alternatively by holding and maneuvering the ultrasonic instrument 18 to treat the target site, while also receiving guidance from the robotic manipulator 16. For instance, the robotic manipulator 16 may be configured, such as at the direction of the surgical navigation system 14, to provide haptic feedback that constrains movement of the ultrasonic instrument 18 as the ultrasonic instrument 18 is moved by the surgeon relative to the target site, such as to promote full treatment of the target site and avoid non-targeted objects. In the telemanipulation mode, the surgeon may control the robotic manipulator 16 to maneuver the ultrasonic instrument 18 using a remote control device, such as a force feedback controller, while also receiving guidance from the remote control device, such as in the form of haptic feedback provided by the remote control device that is similar to that provided in the hands-on mode.

The surgical navigation system 14 may be configured to track the relative poses (i.e., position and orientation) of objects of interest within the surgical workspace. The tracked objects may include, but are not limited to, anatomical structures of the patient P and surgical instruments. The tracked anatomical structures may include at least one volume of patient tissue targeted for treatment and/or at least one volume of patient tissue adjacent the targeted tissue that is not targeted for treatment, and may encompass both soft and hard tissues such as fat, ligaments, nerves, blood vessels, muscle, skin, tumors, and bone. The tracked surgical instruments may include the ultrasonic instrument 18, retractors, other cutting tools, inserters, implants, and waste management devices used during the surgical procedure.

The surgical navigation system 14 may include trackers 20 disposed relative to the objects of interest, and may be configured to cooperate with such trackers 20 to localize the objects of interest in a known coordinate system. For instance, in the example illustrated in FIG. 1, the surgical system 10 is being used to perform a spinal procedure on a patient P, such as a minimally invasive interbody fusion in which an intervertebral disc space is being prepared using the ultrasonic instrument 18 to receive an implant (*e.g.*, spacer, graft, cage). To support preparation of the intervertebral disc space with the ultrasonic instrument 18, the surgical navigation system 14 may be configured to continuously track the pose of each vertebra V adjacent the intervertebral disc space in a known coordinate system using a patient tracker 20A coupled to each of the vertebra V. The surgical navigation system 14 may also be configured to track a pose of the ultrasonic instrument 18 in the known coordinate system via an instrument tracker 20B coupled to the ultrasonic instrument 18. Additionally or alternatively, the surgical navigation system 14 may be configured to track a pose of the ultrasonic instrument 18 via a manipulator tracker 20C coupled to the robotic manipulator 16 as described in more detail below.

In some implementations, the trackers 20 may be optical trackers, in which case the surgical navigation system 14 may be configured to detect light signals transmitted (e.g., emitted or reflected) from each tracker 20 by imaging the tracker 20. The surgical navigation system 14 may then be configured to determine the poses of the trackers 20 in a known coordinate system based on the detected light signals, and thereafter determine the poses of the objects relative to which the trackers 20 are disposed in the known coordinate system based on the determined poses of the trackers 20 and predetermined positional relationships between the objects and trackers 20. The surgical navigation system 14 may have various functions and features as described in U.S. Patent No. 7,725,162 and U.S. Patent Publication No. 2020/0100849, the contents of each which are hereby incorporated by reference herein in their entirety.

While examples are provided in which the surgical navigation system 14 is an optical tracking system, other types of tracking systems may be employed. For instance, in some implementations, the surgical navigation system 14 may be realized as an electromagnetic tracking system in which position sensors are disposed relative to the objects of interest (e.g., fixed to or embedded within the objects). Each position sensor may include a coil that, when moved within an electromagnetic field, generates electrical current in the coil, which may then be communicated to the surgical navigation system 14. This phenomenon may enable the surgical navigation system 14 to determine the location of the coil within a three-dimensional space.

By way of example only, such position sensors may be constructed and operable in accordance with at least some of the teachings of U.S. Pat. No. 8,702,626, the disclosure of which is incorporated by reference herein; U.S. Pat. No. 8,320,711, the disclosure of which is incorporated by reference herein; U.S. Pat. No. 8,190,389, the disclosure of which is incorporated by reference herein; U.S. Pat. No. 8,123,722, the disclosure of which is incorporated by reference herein; U.S. Pat. No. 7,720,521, the disclosure of which is incorporated by reference herein; U.S. Pat. Pub. No. 2014/0364725, the disclosure of which is incorporated by reference herein; U.S. Pat. Pub. No. 2014/0200444, the disclosure of which is incorporated by reference herein; U.S. Pat. Pub. No. 2012/0245456, the disclosure of which is incorporated by reference herein; U.S. Pat. Pub. No. 2011/0060214, the disclosure of which is incorporated by reference herein; U.S. Pat. Pub. No. 2008/0281156, the disclosure of which is incorporated by reference herein; and/or U.S. Pat. Pub. No. 2007/0208252, the disclosure of which is incorporated by reference herein.

Responsive to determining the poses of the objects of interest in a known coordinate system, the surgical navigation system 14 may be configured to display virtual representations of the relative poses of the tracked objects, which may be viewed by the surgeon to aid the surgeon in positioning the ultrasonic instrument 18 relative to the target site and non-targeted objects. For instance, the surgical navigation system 14 may be configured to display images and/or graphical representations (e.g., virtual models) of the objects of interest according to their determined poses. The surgical navigation system 14 may also be configured to control operation of the robotic manipulator 16 and/or the ultrasonic instrument 18 based on virtual objects associated with the tracked objects in the known coordinate system, such as described in PCT Application No. PCT/US2022/054115, U.S. Patent No. 9,775,681, and U.S. Patent Publication No. 2022/0338938, the contents of each of which are hereby incorporated by reference herein in their entirety.

For example, the surgical navigation system 14 may be configured to control the robotic manipulator 16 to maneuver and/or guide movement of the ultrasonic instrument 18 based on a tracked pose of the ultrasonic instrument 18 relative to virtual objects generated in the known coordinate system and associated with the objects of interest, such as to lead the ultrasonic instrument 18 to the target site and/or away from contact with anything beyond the target site (e.g., the non-targeted objects). In this way, the surgical navigation system 14 may both improve surgical accuracy and eliminate damage to surgical instruments caused by unintended contact with other objects, which may result in undesired debris at the target site. Additionally or alternatively, the surgical navigation system 14 may be configured to adjust operation of the ultrasonic instrument 18 based on a tracked pose of the ultrasonic instrument 18 relative the virtual objects in the known coordinate system, such as by ceasing or reducing power to the ultrasonic instrument 18 when the tracked pose indicates the ultrasonic instrument 18 is contacting or near contacting a non-targeted object outside the target site.

Still referring to FIG. 1, the surgical navigation system 14 may include a localizer camera 22 and a navigation cart assembly 24. The navigation cart assembly 24 may house a navigation controller 26 configured to implement the functions, features, and processes of the surgical navigation system 14 described herein. For instance, the navigation controller 26 may be configured to convert optical-based image data received from the localizer camera 22 into tracking data indicative of the relative poses of the tracked objects in the known coordinate system, and to control operation of the robotic manipulator 16 based on the indicated poses as described herein.

The navigation controller 26 may include or be communicatively coupled to a navigation storage 28 of the surgical navigation system 14, and/or may be communicatively coupled to a user interface 30A of the surgical navigation system 14. The navigation storage 28 may store data for facilitating operation of the surgical navigation system 14, as described in more detail below. The user interface 30A may facilitate user interaction with the surgical navigation system 14 and navigation controller 26. For example, the user interface 30A may include one or more output devices that provide information to a user, such as from the navigation controller 26. The output devices may include a display 32 adapted to be situated outside of a sterile field including the surgical workspace, and may include a display 34 adapted to be situated inside the sterile field. The displays 32, 34 may be adjustably mounted to the navigation cart assembly 24. The user interface 30A may also include one or more input devices that enable user-input to the surgical navigation system 14 and the navigation controller 26. For instance, the input devices may include a keyboard, mouse, and/or touch screen 36 that can be interacted with by a user to input operating parameters to and control aspects of the navigation controller 26. The input devices may also include a microphone that enables user input through voice-recognition technology.

The localizer camera 22 may be configured to facilitate identification of the poses of the tracked objects relative to a known coordinate system, such as a localizer coordinate system LCLZ of the localizer camera 22, by generating optical-based image data indicating poses of the trackers 20 disposed relative to the objects of interest in the known coordinate system. The localizer camera 22 may have an outer casing 38 that houses at least two optical sensors 40. Each of the optical sensors 40 may be adapted to detect light signals of a particular frequency band that are transmitted from the trackers 20, such as nonvisible light signals (e.g., infrared or ultraviolet). While FIG. 1 illustrates the localizer camera 22 as a single unit with multiple optical sensors 40, in an alternative example, the localizer camera 22 may include separate units arranged around the surgical workspace, each with a separate outer casing 38 and one or more optical sensors 40.

The optical sensors 40 may be one-dimensional or two-dimensional charge-coupled devices (CCDs). For example, the outer casing 38 may house two two-dimensional CCDs or three one-dimensional CCDs for triangulating the positions of optical markers of the trackers 20 in the known coordinate system, described in more detail below. Additionally or alternatively, the localizer camera 22 may employ other optical sensing technologies, such as complementary metal-oxide semiconductor (CMOS) active pixels.

The localizer camera 22 may be mounted to an adjustable arm to selectively position the optical sensors 40 with a field of view of the surgical workspace and target site that, ideally, is free from obstacles. The localizer camera 22 may be adjustable in at least one degree of freedom by rotating about a rotational joint, and may be adjustable about two or more degrees of freedom.

For each object of interest in the surgical workspace desired to be tracked, a tracker 20 may be disposed relative to the object according to a known positional relationship, such that a pose of the object in the known coordinate system may be inferred from a determined pose of the tracker 20 in the known coordinate system. A tracker 20 disposed in the surgical workspace for tracking a given object may be considered as associated with the given object. In some examples, a given tracker 20 may be directly affixed to the object of interest associated with the tracker 20. In some examples, the object to which a given tracker 20 is affixed may be rigid and inflexible so that movement of the object cannot or is unlikely to alter the positional relationship between the object and the tracker 20. In other words, the relationship between the tracker 20 and the object to which the tracker 20 is attached may remain fixed, notwithstanding changes in the position of the object within the surgical workspace.

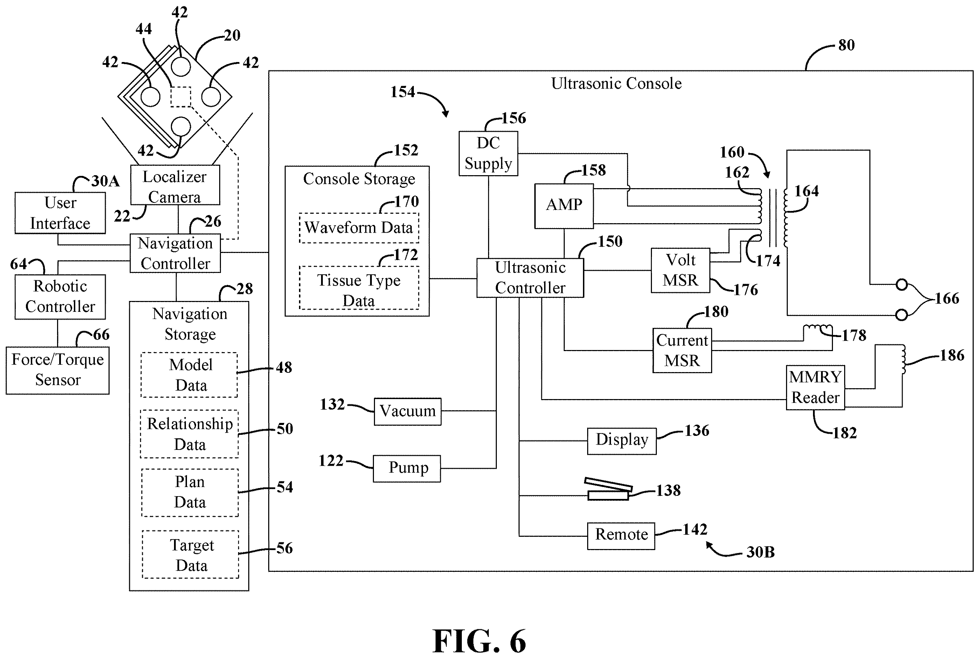

Referring to FIG. 6, each tracker 20 may include a known arrangement of at least three optical markers 42 that direct light signals to the optical sensors 40 of the localizer camera 22. In some implementations, the known arrangement of markers 42 of each tracker 20 may be unique to facilitate differentiation of the trackers 20 by the surgical navigation system 14. In some implementations, the markers 42 of each tracker 20 may be realized as active markers that receive an electrical current from a power source to generate and emit light signals to the optical sensors 40. In this case, the trackers 20 may each be powered by an internal battery, or may have leads to receive power through the navigation controller 26. For instance, the markers 42 may be light emitting diodes (LEDs) that emit light, such as nonvisible light (e.g., infrared or ultraviolet light), towards the optical sensors 40.

Each tracker 20 may also include a tracker controller 44 communicatively coupled to the markers 42 and to the navigation controller 26. The tracker controller 44 of each tracker 20 may be configured to control the rate and order in which the markers 42 of the tracker 20 are triggered to emit light, such as at the direction of the navigation controller 26. For example, the tracker controller 44 of each tracker 20 may cause the markers 42 of the tracker 20 to emit light at different rates and/or times to facilitate differentiation of the markers 42 by the navigation controller 26. In some examples, the navigation controller 26 may form a bidirectional infrared communication channel with the tracker controller 44 of each tracker 20 to control the timing of the triggering of the active markers 42 operated by the tracker controller 44, write data to and/or read data from a storage device of the tracker 20, and/or get a status (e.g., battery level, broken LEDs) of the tracker 20 or the object to which the tracker 20 is affixed.

In some implementations, the markers 42 of one or more the trackers 20 may be realized as passive markers, such as reflectors that reflect light emitted from the localizer camera 22. To this end, the localizer camera 22 may include a light source 46 (FIG. 1) that illuminates the trackers 20 with light, such as nonvisible light (e.g., infrared or ultraviolet). The markers 42 may then be configured to reflect the light back towards the localizer camera 22 for detection by the optical sensors 40.

Prior to the start of a surgical procedure, pre-operative medical images may be generated for anatomical objects of interest, such as anatomical structures defining and/or adjacent a target site. For example, when the volume of patient tissue targeted for treatment is in the patient's spine area, pre-operative images of the patient's spinal column may be taken. These images may be based on MRI scans, radiological scans, or computed tomography (CT) scans of the patient's anatomy, and may be used to develop virtual models of the anatomical structures, stored as model data 48 in the navigation storage 28. Also prior to the surgical procedure, the navigation controller 26 may receive and store as model data 48 virtual models for other tracked objects of interest, such as the ultrasonic instrument 18, the robotic manipulator 16, and/or the trackers 20.

Each virtual model for a given object may include a three-dimensional model (e.g., point cloud, mesh, CAD) of the object, and/or may indicate coordinates in a three-dimensional coordinate system specific to the object that correspond to the relative positions of features of the object. For instance, a virtual model for a given tracker 20 may indicate coordinates in a three-dimensional coordinate system specific to the tracker 20 that correspond to the relative positions of markers 42 of the tracker 20. As a further example, a virtual model for a given surgical instrument may indicate coordinates in a three-dimensional coordinate system specific to the surgical instrument that correspond to the relative positions of features of the housing and/or end effector of the surgical instrument. As a further example, a virtual model for an anatomical structure may indicate coordinates in a three-dimensional coordinate system specific to the anatomical structure that correspond to the relative positions of specific features of the anatomical structure and/or that define at least a portion of a target site of the patient.

A virtual model for a given anatomical structure may also indicate the type(s) of tissue(s) forming the anatomical structure, such as by indicating the stiffness or density of such tissue(s). In some examples, such tissue type(s) may be expressed in the model data 48 as expected electrical characteristics of a drive signal supplied to the ultrasonic instrument 18 when vibrating against the tissue.

In some implementations, the navigation controller 26 may be configured to apply a segmentation algorithm to the medical images to generate the virtual models for the anatomical structures of interest. The segmentation algorithm may be configured to employ various technologies in machine vision to determine boundaries associated with various objects and/or tissue types within the image. In some examples, the segmentation algorithm may be configured to receive as inputs the medical image and the type of procedure to be performed, and apply one or more of edge detection, clustering, region growing, level set evolution (e.g., fast marching segmentation), and other segmentation algorithms calibrated based on the type of procedure to the medical image to identify boundaries between various objects of interest within the image. For discectomies as an example, the disc space between two adjacent vertebrae can be captured automatically such as by using a deep learning approach to identify the vertebral endplates and define the disc space between two adjacent vertebrae as the convex hull of the inferior endplate of the superior vertebra and the superior endplate of the inferior endplate. Zones of risk corresponding to non-targeted objects can also be automatically identified. For some procedures, automatic screw planning capabilities may also be implemented that provide trajectories for drill paths of the ultrasonic instrument 18.

In addition or alternatively, a user may be able to interact with the user interface 30A of the surgical navigation system 14 to manually identify boundary(s) within the images and/or to manipulate the boundary(s) generated by the segmentation algorithm described above. For instance, adjustable planes with definable regions of interest can be provided to define cuts such as for a laminectomy or for a pedicle subtraction osteotomy.

In addition or alternatively to processing pre-operative medical images, the above-described virtual models may be generated from kinematic studies, bone tracing, and other methods.

Further prior to the start of the surgical procedure, the navigation controller 26 may receive and store relationship data 50 defining the predetermined positional relationships, also referred to as spatial registrations, between the trackers 20 and the objects associated with the trackers 20. Each spatial registration may define a pose of a given tracker 20, or more particularly a pose of the markers 42 of the tracker 20, relative to the object associated with tracker 20, such as by reference to the virtual models and/or coordinate systems of the object and tracker 20. For instance, in the example illustrated in FIG. 1, the spatial registration for each tracker 20A may indicate the pose of a coordinate system of the vertebra V to which the tracker 20A is affixed within the coordinate system of the tracker 20A, or vice versa. Thus, responsive to identifying a pose of a tracker 20A, and correspondingly the pose of the coordinate system associated with the tracker 20A, relative to the known coordinate system, the navigation controller 26 may reference the relationship data 50 for the tracker 20A to determine the pose of the coordinate system of the vertebra V, and correspondingly the pose of the vertebra V, relative to the known coordinate system.

The spatial registration between a given tracker 20 and an object associated with the given tracker 20 may be determined by known registration techniques, such as point-based registration in which a calibration tool 52 (FIG. 1) including an arrangement of markers 42 is used to touch off points across the object being registered. By monitoring the position of the distal end of the calibration tool 52 via its markers 42 relative to that of the tracker 20 in the known coordinate system (e.g., the localizer coordinate system LCLZ) as the distal end of the calibration tool 52 is touched off points of the object, the navigation controller 26 may be configured to determine a pose of the object relative to the tracker 20. Such data may also be used by the navigation controller 26 to generate a virtual model of the object. In addition or alternatively, the navigation controller 26 may be configured to determine the spatial registration of a given tracker 20 relative to an associated object such as an anatomical structure from a medical image depicting both a pose of the tracker 20 and the anatomical structure in a same coordinate system. In some implementations, the spatial registration for a tracker 20 associated with a given surgical instrument may have been previously measured during coupling of the surgical instrument and tracker 20 at a previous point in time.

Referring again to FIG. 6, the navigation controller 26 may also receive and store a surgical plan 54 and target data 56 prior to a procedure. The surgical plan 54 may identify the patient anatomical structures involved in the surgical procedure, tissue volumes targeted for resection, non-targeted objects which should be avoided such as due to the presence of critical structures, material and/or densities of the target tissue and/or non-targeted objects, instruments and instrument geometries to be used in the procedure, and planned trajectories of instruments and the planned movements of patient tissue during the surgical procedure.

The target data 56 may indicate one or more target parameters for sensing and/or resecting patient tissue using the ultrasonic instrument 18, and may be ultrasonic instrument 18 specific. For instance, the target data 56 may indicate a target force to apply on the ultrasonic instrument 18 relative to contacted patient tissue, a target pose for the ultrasonic instrument 18 relative to contacted patient tissue, and/or a target velocity for moving the ultrasonic instrument 18 relative to contacted patient tissue for sensing the contacted tissue with the ultrasonic instrument 18, and may also include one or more of the same items for resecting the contacted tissue with the ultrasonic instrument 18. During a procedure, the robotic manipulator 16, such as at the direction of the navigation controller 26, may be configured to regulate movement of the ultrasonic instrument 18 according to the target parameters, which may provide more repeatable and consistent resections and sensing relative to a manual approach.

In some implementations, the target data 56, such as the target forces and/or velocities, may be further optimized taking into account the type of tissue being contacted or indicated as being contacted by the tracking data. For instance, as bone density correlates to CT Hounsfield units, the navigation controller 26 may be configured to generate a tissue-density map from a received pre-operative CT scan in the known coordinate system in which the ultrasonic instrument 18 is also tracked, and determine target forces for the ultrasonic instrument 18 based on the tracked pose of the ultrasonic instrument 18 relative to the tissue-density map, or in other words, based on the density of the tissue indicated as being contacted by the ultrasonic instrument 18. This may yield superior cutting and/or sensing, especially given the high variability of bone densities observed among multiple patients (e.g., osteoporotic bone vs "healthy" bone). In some implementations, the target data 56 may also include varying predefined motion patterns to apply to the ultrasonic instrument 18 during tissue resection for different tips 82 and/or different types of contacted tissue, such as to simulate and/or accentuate an expected tactile feel corresponding to resection of a given type of tissue and/or with a given tip 82.

During a surgical procedure, the navigation controller 26 may be configured to operate the localizer camera 22 to cause the optical sensors 40 to generate optical-based signals indicative of detected light signals received from the trackers 20, or more particularly indicative of the image plane positions of the optical sensors 40 in which such light signals were detected. The sampling rate of the optical sensors 40 is the rate at which the optical sensors 40 detect light signals from sequentially triggered markers 42. The optical sensors 40 may have sampling rates of 100 Hz or more, or more preferably 300 Hz or more, or most preferably 500 Hz or more. In one instance, the optical sensors 40 may have sampling rates of 8000 Hz.

Responsive to the optical sensors 40 receiving light signals from the trackers 20, the optical sensors 40 may output optical-based signals indicating the positions of the markers 42 of the trackers 20 relative to the localizer camera 22, which may generally be used by the navigation controller 26 to determine poses of the objects associated with the trackers 20 relative to the localizer camera 22. In particular, each optical sensor 40 may include a one- or two-dimensional sensor area (also referred to as an "image plane") that detects light signals from the trackers 20, and responsively outputs optical-based signals indicating pixel coordinates within the sensor area that each light signal was detected. The optical-based signals output from each optical sensor 40 may thus represent an image of the trackers 20 generated by the optical sensor 40 from the detected light signals, with the image including artefacts in pixel coordinates corresponding to the positions in the image plane of the optical sensor 40 that light signals were detected. The detected position of each light signal may be based on the angle at which the light signal is received by the optical sensor 40, and may thus correspond to the position of the marker 42 in the surgical workspace that transmitted the detected light signal towards the optical sensor 40.

The localizer camera 22 may be configured to generate image data based on the optical-based signals received from the optical sensors 40, and communicate such optical-based image data to the navigation controller 26. The optical-based image data may indicate the image and/or image plane positions represented by the optical-based signals received from each optical sensor 40.

The navigation controller 26 may be configured to generate tracker pose data indicating the poses of the trackers 20 relative to the localizer camera 22 based on the received image data. More particularly, the navigation controller 26 may determine a position of each marker 42 in the localizer coordinate system LCLZ based on the image data. For instance, the navigation controller 26 may be configured to correlate artefacts corresponding to a same optical marker 42 in the image data, triangulate the positions of the optical markers 42 relative to the localizer camera 22 based on the positions of the correlated artefacts in the image data and a known positional relationship between the optical sensors 40, and assign the triangulated positions to the markers 42 of each tracker 20 based on a known geometry of the markers 42 of each tracker 20, or alternatively based on a known frequency, timing, or intensity associated with each marker 42. The positions of the markers 42 in the localizer coordinate system LCLZ may define the poses of the trackers 20 in the localizer coordinate system LCLZ.

Based on the poses of the trackers 20 in a known coordinate system, the navigation controller 26 may be configured to generate tracking data indicating the poses of the objects associated with the trackers 20 in the known coordinate system. Specifically, the navigation controller 26 may apply the previously stored relationship data 50 indicating the positional relationships between the trackers 20 and the objects to which the trackers 20 are associated to the tracker pose data to determine the poses of the associated objects in the known coordinate system.

Additionally or alternatively to tracking objects of interest such as anatomical structures in a known coordinate system via trackers 20 disposed relative to the objects, the navigation controller 26 may be configured to localize one or more objects in the known coordinate system based on a pose of each object in one or more medical images generated by an imaging system prior to and/or during the procedure, and a pose of the imaging system relative to the known coordinate system when the medical images are generated. To this end, in some implementations, the surgical system 10 may include an imaging system coupled to the navigation controller 26 and configured to generate medical images depicting one or more of the objects of interest, such as that described in PCT Application No. PCT/US2023/017790, the contents of which are hereby incorporated by reference herein in their entirety. The imaging system may include a tracker 20 having a predetermined spatial registration to the pixel coordinates of the medical images generated by the imaging system, which may also be stored by the navigation controller 26 as relationship data 50. In other words, the relationship data 50 may indicate a pose of a coordinate system of the medical images relative to a coordinate system of the imaging system tracker 20.

Thus, prior to and/or during a procedure, the navigation controller 26 may receive medical images from the imaging system, and determine poses of the tracker 20 coupled to the imaging system that correspond to the images. The navigation controller 26 may then segment each medical image as described above to determine a pose of one or more objects of interest in the coordinate system of the medical image, and thereafter determine the pose of such objects in the known coordinate system, such as the localizer coordinate system LCLZ, based on the determined pose of the tracker 20 coupled to the imaging system relative to the known coordinate system and the predetermined spatial registration between the tracker 20 and the medical image.

Referring again to FIG. 1, the ultrasonic instrument 18 may form part of an end effector of the robotic manipulator 16. The robotic manipulator 16 may include a base 58, several links 60 extending from the base 58, and several active joints 62 for moving the ultrasonic instrument 18 with respect to the base 58. The links 60 may form a serial arm structure as shown in FIG. 1, a parallel arm structure, or other suitable structure.

Similar to the surgical navigation system 14, the robotic manipulator 16 may house a robotic controller 64 configured to implement the functions, features, and processes of the robotic manipulator 16 described herein. During a surgical procedure, the robotic controller 64 may be configured to determine a desired location to which the ultrasonic instrument 18 should be moved, such as based on instructions received from the navigation controller 26 and/or the surgeon. Based on this determination, and information relating to the current position of the ultrasonic instrument 18, the robotic controller 64 may be configured to determine an extent to which the links 60 need to be moved to reposition the ultrasonic instrument 18 from the current position to the desired position. Data indicating where the links 60 are to be repositioned may be forwarded to joint motor controllers (e.g., one for controlling each motor) that control the active joints 62 of the robotic manipulator 16. Responsive to receiving such data, the joint motor controllers may be configured to move the links 60 in accordance with the data, and consequently move the ultrasonic instrument 18 to the desired position.

As mentioned above, a separate tracker 20C may be attached to the base 58 of the robotic manipulator 16 to track a pose of the base 58 with the localizer camera 22. In some implementations, such as instead of or in addition to utilizing the instrument tracker 20B, the surgical navigation system 14 may be configured to track the pose of the ultrasonic instrument 18 based on a tracked pose of the base 58 in the known coordinate system and positions of the joints 62. More specifically, during a registration workflow, such as executed by the navigation controller 26, the links 60 of the robotic manipulator 16 may be adjusted, such as at the direction of a user interacting with the robotic manipulator 16, to position an end-effector of the robotic manipulator 16 to a certain number of defined non-coplanar landmarks with known positions in the known coordinate system while the surgical navigation system 14 tracks the pose of the base 58 and receives encoder data from the robotic controller 64 indicative of the positions of the joints 62. The positions of the landmarks in the known coordinate system together with the tracked pose of the base 58 in the known coordinate system and encoder positions may be used by a point-cloud-match algorithm, such as Arun's method for 3D registration or an Iterative Closest Point algorithm (ICP), executed by the surgical navigation system 14 to generate an estimation model of a transformation between the base 58 and position of the end effector in the known coordinate system as a function of the positions of the joints 62. The pose of the ultrasonic instrument 18 attached on the end-effector of the robotic manipulator 16 may then be calibrated to the pose of the end-effector, such as by touching the distal region 84 of the tip 82 to a tracked reference body with known geometry (e.g., calibration device), which may correspondingly enable the navigation controller 26 to transform a determined pose of the base 58 coordinate system in the known coordinate system to a pose of the ultrasonic instrument 18 coordinate system in the known coordinate system.

In some implementations, the robotic manipulator 16 may further include at least one force-torque sensor 66 configured to generate data indicative of forces being applied to the ultrasonic instrument 18 by the surgeon and/or robotic manipulator 16. For instance, the force-torque sensor 66 may incorporate multiple strain gauges configured to measure forces being applied to the ultrasonic instrument 18 in various directions, such as in 6 degrees of freedom ("DOF"). As shown in the illustrated example, the robot flange of the robotic manipulator 16 may incorporate the force-torque sensor 66. Alternatively, each joint 62 of the robotic manipulator 16 may be equipped with a force-torque sensor 66. During operation of the surgical system 10, the robotic controller 64 may communicate with the force-torque sensor(s) 66 to determine one or more forces being applied to the ultrasonic instrument 18. The robotic controller 64 may forward such force data to the navigation controller 26 and/or ultrasonic controller 150 (FIG. 6) for various processes described in more detail below.

Using the tracking data indicative of the relative poses of the objects of interest, the navigation controller 26 may be configured to control operation of the robotic manipulator 16, and/or control operation of the ultrasonic instrument 18, so as to treat the target site while limiting unintentional treatment of non-targeted objects, such as non-targeted tissue and other surgical instruments near the target site. To this end, the navigation controller 26 may be configured to associate virtual objects with the tracked objects in the known coordinate system, and adjust the poses of the virtual objects relative to the known coordinate system throughout the surgical procedure to correspond to the tracking data. For instance, the navigation controller 26 may be configured to associate at least one virtual object with each target site of patient tissue targeted for treatment, and/or associate at least one virtual object with each non-targeted object adjacent a target site.

Each virtual object associated with a given tracked object may be defined based on a virtual model developed for the object as described above. At least one of the associated virtual objects may correspond to a boundary of one of the tracked objects, such as a boundary of a target site and/or non-targeted object. Such virtual object(s) may define one or more virtual boundaries across which a virtual object associated with the ultrasonic instrument 18 is to stay within during treatment of the target site. The surgical navigation system 14 may thus be configured, such as when the robotic manipulator 16 is being operated in the autonomous mode, to control the robotic manipulator 16 to maneuver the ultrasonic instrument 18 to treat the target site while avoiding contact between a virtual object associated with the ultrasonic instrument 18 with the virtual boundaries defined by the other virtual objects.

Additionally or alternatively, at least one of the associated virtual objects may be a haptic object that generates a force (e.g., positive or negative) on the robotic manipulator 16 based on a tracked pose of the ultrasonic instrument 18 relative to the haptic object, which may be felt by the surgeon in the hands-on or telemanipulation modes so as to guide the surgeon in maneuvering the ultrasonic instrument 18 to treat the target tissue. For instance, the haptic objects may include virtual magnets that guide movement of the ultrasonic instrument 18 with force feedback along a planned path, and/or may include virtual fixtures that resist movement of the ultrasonic instrument 18 from deviating from the planned path by being configured to cause an increase in the stiffness of the ultrasonic instrument 18 when the ultrasonic instrument 18 is moved towards the fixture. To this end, each haptic virtual object may include a control parameter configured to either attract or resist movement of the ultrasonic instrument 18 towards the virtual object, such as based on whether or not the virtual object is associated with tissue targeted for resection or a non-targeted object. Non-limiting examples of haptic virtual objects and corresponding operation of the robotic manipulator 16 based on such objects are described in U.S. Patent Nos. 9,775,681 and 10,398,449, the contents of each of which are hereby incorporated by reference herein in their entirety.

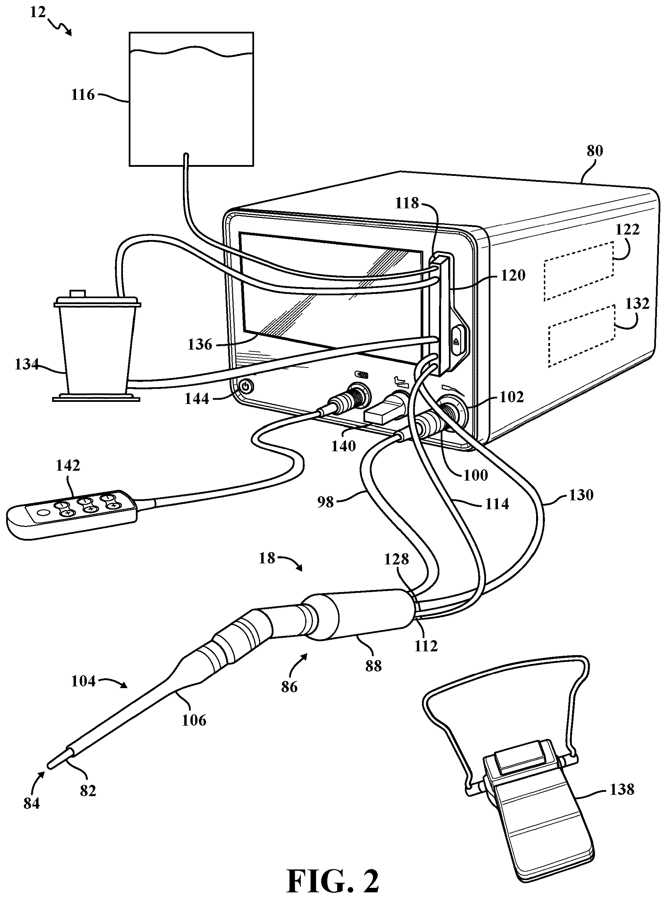

For instance, in some implementations, repulsive haptic objects may be enforced using a linear spring model or a quadratic model. In the linear spring model, the amount of resistive force applied by the robotic manipulator 16 on movement of the ultrasonic instrument 18 may increase with decrease of the distance between a distal region 84 of the tip 82 (FIG. 2) of the ultrasonic instrument 18 and the virtual object according to a linear relationship, such as indicated by the tracking data generated by the navigation controller 26. In the quadratic model, the amount of resistive force applied by the robotic manipulator 16 on movement of the ultrasonic instrument 18 may increase with decrease of the distance between the distal region 84 of the tip 82 of the ultrasonic instrument 18 and the virtual object according to a quadratic relationship.

For instance, the amount of force $F^*$ to constrain the distal region 84 of the tip 82 of the ultrasonic instrument 18 within the target site may be calculated, such as by the surgical navigation system 14 or the robotic manipulator 16, as follows:

$$F^* = \begin{cases} 0, & \rho > \rho_0 \\ \gamma \left(\frac{1}{\rho} - \frac{1}{\rho_0}\right) \frac{1}{\rho^2} \nabla\rho, & \rho \leq \rho_0 \end{cases}$$

**where** $\rho$ is the Euclidean distance between the distal region 84 of the tip 82 to the virtual object, $\rho_0$ is a minimum distance between the distal region 84 of the tip 82 for the virtual object to apply a force on the ultrasonic instrument 18, and $\nabla\rho$ is the partial derivative in the direction of a next cell or region the surgical workspace, which may prevent the ultrasonic instrument 18 being moved into a repulsive virtual object. The robotic manipulator 16 may then be controlled, such as by the surgical navigation system 14, based on calculated $F^*$ by calculating target torques $\tau$ for joints 62 of the robotic manipulator 16 as follows:

$$\tau = J^T * (\lambda * F^* * \mu * p) + N * \tau\_ns$$

where $J$ is the Jacobian matrix of the robotic manipulator 16 end effector, $\lambda$ is the inertia matrix of the robotic manipulator 16 expressed in the end-effector frame, $\mu$ is the Coriolis vector and centrifugal forces, $p$ is the gravity force vector of the end effector of the robotic manipulator 16, $N$ is the nullspace projection, and $\tau\_ns$ is the joint torques in the nullspace.

Referring now to FIG. 2, the ultrasonic system 12 may include a control console 80 and the ultrasonic instrument 18 coupled to the control console 80. The ultrasonic instrument 18 may include a tip 82 with a distal region 84 configured for contacting and treating patient tissue. During operation, the control console 80 may be configured to generate and source an AC drive signal to the ultrasonic instrument 18 that induces ultrasonic energy in the ultrasonic instrument 18, and in turn causes the distal region 84 of the tip 82 to rapidly vibrate. An operator may then position the distal region 84 (also referred to as the tip head 84) of the vibrating tip 82 against patient tissue to resect the contacted tissue. The frequency, amplitude, and velocity of the vibrations of the tip 82 may correspond to that of the sourced AC drive signal.

The ultrasonic instrument 18 may include a handpiece 86 for being grasped by an operator to maneuver the ultrasonic instrument 18 against patient tissue. The tip 82 may be removably coupled to the handpiece 86 so as to enable the handpiece 86 to be used with different interchangeable tips 82. Different tips 82 removably coupleable to the handpiece 86 may be configured for different types of procedures. For instance, some tips 82 removably coupleable to the handpiece 86 may be configured for ablating soft tissue, such as by inducing cavitation in such tissue. A tip 82 configured for ablating soft tissue may define a lumen for providing suction at the surgical site through the tip 82. Some tips 82 removably coupleable to the handpiece 86 may be configured for ablating hard tissue such as fibrous tissue and bone. A tip 82 configured for ablating hard tissue may feature a tip head 84 formed with teeth or flutes dimensioned to remove tissue via a cutting action. Tips 82 removably coupleable to the handpiece 86 may also be of different lengths for providing access to patient anatomy at different depths.

Some tips 82 removably coupleable to the handpiece 86 may be designed to vibrate in a longitudinal mode in which the tip head 84 exhibits substantially longitudinal vibrations only. Other tips 82 removably coupleable to the handpiece 86 may additionally or alternatively be designed to vibrate in a longitudinal and torsional mode in which the tip head 84 simultaneously exhibits longitudinal and torsional vibrations, and/or a torsional mode in which the tip head 84 exhibits substantially torsional vibrations only, and/or a flexural mode in which the tip head 84 exhibits substantially flexural vibrations only, and/or a combination flexural mode in which the tip head 84 exhibits flexural vibrations simultaneously with longitudinal and/or torsional vibrations.

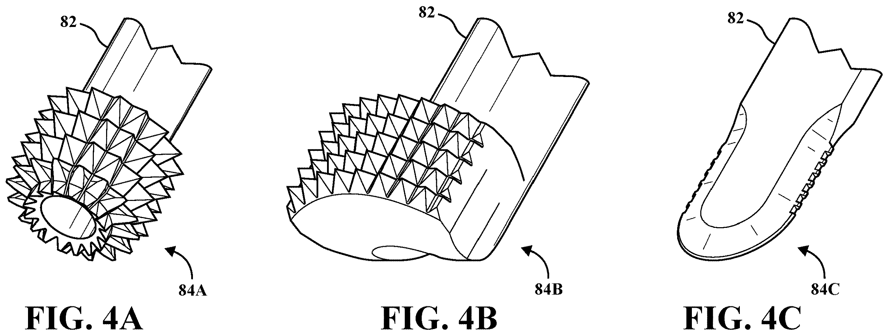

FIGS. 4A-4C illustrate varying tip heads 84A-C that may be formed with different tips 82 removably coupleable to the handpiece 86. FIG. 4A illustrates a tip head 84A having a radially symmetric configuration such that effective use of the tip for resecting and/or sensing contacted patient tissue is relatively less dependent on the pose of the tip head 84 relative to the patient tissue. Conversely, FIGS. 4B and 4C illustrate radially asymmetric tip heads 84B, 84C such that the effective use of the tip for resecting and/or sensing contacted patient tissue is relatively more dependent on the pose of the tip head 84 relative to the patient tissue. Thus, when a tip 82 with an asymmetric tip head 84 is currently coupled to the handpiece 86, the surgical navigation system 14 may be configured to determine a target resection pose and/or a target sense pose specific to the tip 82 for resecting and/or sensing contacted patient tissue respectively, and influence control of the robotic manipulator 16 to position the tip head 84 according to the target resection pose and/or target sense pose when resecting and/or sensing patient tissue respectively.

Referring again to FIG. 2, the handpiece 86 may form a proximal end of the ultrasonic instrument 18, and the tip 82 coupled to the handpiece 86 may form a distal end of the ultrasonic instrument 18. "Proximal" may be understood as towards an operator holding the ultrasonic instrument 18 and away from the tissue to which the tip 82 is being applied, and "distal" may be understood as away from the operator and towards the tissue to which the tip 82 of the ultrasonic instrument 18 is being applied.

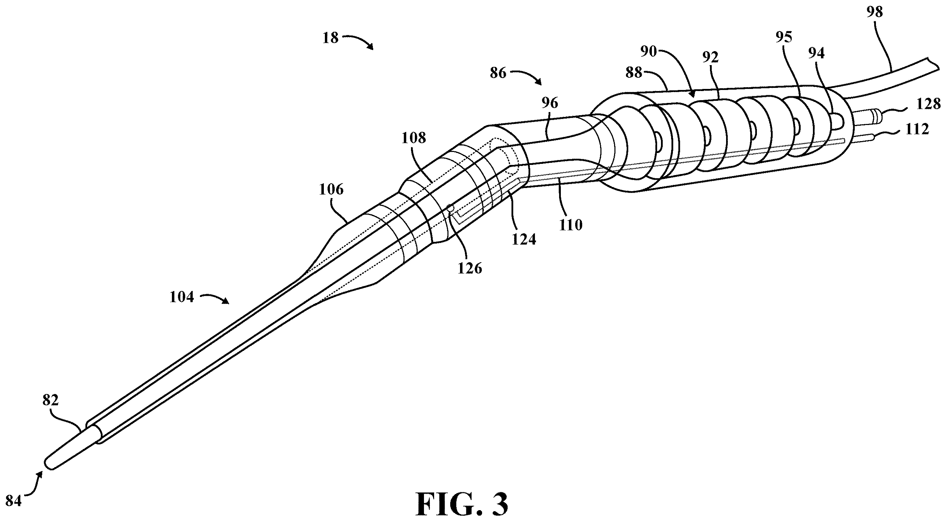

Referring now to FIG. 3, the handpiece 86 may include a housing 88 that defines a handle for the operator to grasp and maneuver the ultrasonic instrument 18 and may also include a transducer 90 disposed in a void defined by the housing 88. The transducer 90 may include one or more drivers 92, such as piezoelectric crystals. The drivers 92 may be disc shaped and may be arranged within the housing 88 end to end in a stack. Each driver 92 may be formed from a material that, upon application of an alternating electrical current, undergoes momentary expansions and contractions along the longitudinal axis of the driver 92, namely, the axis that extends between the proximally and distally directed faces of the driver 92. Insulating discs may be disposed between and tightly abut adjacent drivers 92. The transducer 90 may further include a tube 94, which may extend through the collinear longitudinal axes of the drivers 92, which in turn may form an axis aligned with a longitudinal axis of the tube 94.

The handpiece 86 may also include a proximal end mass 95 attached to or adjacent the proximally directed face of the most proximally located driver 92. The exposed proximal end section of the tube 94 may be fixedly attached to the proximal end mass 95. If the tube 94 is threaded, then the proximal end mass 95 may be a nut.

The handpiece 86 may also include a horn 96 at least partially disposed within the void defined by housing 88. The horn 96 may be coupled to the distal end of the transducer 90. The horn 96 may be constructed from a rigid steel alloy, titanium, aluminum or similar material. In operation, as the transducer 90 expands and contracts, the horn 96 may oscillate. The horn 96 may be removably coupled to the transducer 90. For example, the proximal end of the horn 96 may include a threaded male coupler and the distal end of the transducer 90 may include a corresponding female threaded coupler. Alternatively, the transducer 90 and the horn 96 may be permanently coupled via a weld, adhesive, or similar bonding process.

The tip 82 may be removably couplable to the horn 96. More specifically, the distal end of the horn 96 may include a threaded coupler configured to engage corresponding threads on the proximal end of the tip 82. It is further contemplated that other coupling methods may be utilized to removably couple the tip 82 to the horn 96. For example, the distal end of the horn 96 may comprise features that allow snap fit engagement with the tip 82.

Referring again to FIG. 2, the ultrasonic instrument 18 may be removably couplable to the control console 80 via a power cable 98, also referred to as an electrical cable herein. One end the electrical cable 98 may be permanently connected to the proximal end of the housing 88 of the ultrasonic instrument 18, and the other end of the electrical cable 98 may include an adapter 100 corresponding to a socket 102 of the control console 80. The socket 102 may be shaped to receive the adapter 100, and may include electrical contacts corresponding to electrical contacts of the adapter 100 such that when the adapter 100 is fully seated in the socket 102, an electrical connection is formed between the ultrasonic instrument 18 and the control console 80.

Upon actuation of the ultrasonic instrument 18, the control console 80 may generate and source an AC drive signal to the ultrasonic instrument 18 over the electrical cable 98. The ultrasonic instrument 18 may be designed so that the AC drive signal from the control console 80 is applied to each of the drivers 92 of the transducer 90 in parallel, which may cause the drivers 92 to simultaneously expand and contract along a longitudinal axis of the transducer 90 in accordance with the AC drive signal. The stack of drivers 92 may be between 1 and 5 cm in length. The distance, or amplitude, of movement over a single expansion/contraction cycle of the drivers 92 may be between .01 and 10 microns.

The horn 96 may be configured to amplify this movement. Consequently, the distal end of the horn 96 and, by extension, the tip 82, may each move back and forth along its longitudinal axis between a fully contracted position to a fully extended position, thereby producing a longitudinal vibrating motion. In some examples, the maximum peak-to-peak vibration of the tip head 84, representing a single movement from the fully contracted position to the fully extended position, may be 1000 microns, or 500 microns, or 300 microns.

As previously described, some tips 82 removably coupleable to the handpiece 86 may be configured to exhibit both longitudinal and torsional vibrations, and/or substantially torsional vibrations, and/or flexural vibrations at their tip heads 84. Such a tip 82 may include a feature along its length, such as helical grooves, that is configured to convert at least a portion of the longitudinal vibrations applied to the proximal end of the tip 82 into vibrations at the tip head 84 having both a longitudinal component and a torsional component and/or having substantially only a torsional component and/or a flexural component.

Referring now to both FIGS. 2 and 3, to assist in reducing heat generation during a procedure, the ultrasonic instrument 18 may define an irrigation pathway for supplying irrigating fluid to the distal region 84 of the tip 82 and the surgical site. To this end, the ultrasonic instrument 18 may include an irrigation sleeve 104 adapted to be disposed around the tip 82 and removably coupled to the handpiece 86, such as the housing 88 of the handpiece 86, for supplying irrigating fluid to at least the distal region 84 of tip 82 and the surgical site.

The irrigation sleeve 104 may include a sleeve body 106 having open proximal and distal ends and defining a lumen 108 extending between the open proximal and distal ends. The sleeve body 106 may be adapted to be coupled to the handpiece 86, such as the housing 88 of the handpiece 86, so that the tip 82 extends through the lumen 108 and out the open distal end of the sleeve body 106. For instance, the proximal end of the sleeve body 106 may be formed with a coupling feature for releasably coupling the sleeve body 106 to the distal end of the housing 88. When disposed over the tip 82 and coupled to the housing 88, the sleeve body 106 may be radially spaced from the tip 82 and may be spaced longitudinally away from the tip head 84 as described above. The components of the ultrasonic instrument 18 may be dimensioned so that during normal operation, the tip 82 does not contact the irrigation sleeve 104.

During operation of the ultrasonic instrument 18, irrigating fluid may flow from the handpiece 86, into the gap between the tip 82 and the sleeve body 106, and then out the open distal end of the sleeve body 106. More specifically, the handpiece 86 may include an irrigation conduit 110 running through the housing 88 from the proximal end to the distal end of the handpiece 86. The proximal end of the irrigation conduit 110 may be coupled to a fitting 112 of the ultrasonic instrument 18 that extends from a proximal end of the handpiece 86 for receiving an irrigation line 114. The irrigation line 114 may be coupled to a fluid supply 116 via a cassette 118, which may be configured to be inserted into a corresponding slot 120 of the control console 80. During operation of the ultrasonic system 12, a pump 122 of the control console 80 may operate on the cassette 118 to draw fluid from the fluid supply 116 into the irrigation line 114 and thereafter into the irrigation conduit 110.

The irrigation sleeve 104 may similarly include an irrigation conduit 124 in fluid communication with the lumen 108 defined by the sleeve body 106. The irrigation conduit 124 may extend from the proximal end of the sleeve body 106 and run adjacent the lumen 108 to an aperture 126 formed in a wall of the lumen 108. The aperture 126 may be positioned at an intermediary position between the proximal and distal ends of the lumen 108 and may be configured to supply irrigating fluid from the irrigation conduit 124 into the gap between the tip 82 and the sleeve body 106. The proximal end of the irrigation conduit 124 of the irrigation sleeve 104 may be adapted to fluidly engage the distal end of the irrigation conduit 110 of the handpiece 86 when the irrigation sleeve 104 is coupled to the handpiece 86.

Accordingly, during operation of the ultrasonic instrument 18, irrigating fluid may flow from a fluid supply 116, through the irrigation line 114, fitting 112, and conduits 110, 124, and out the aperture 126 into the lumen 108. Such irrigating fluid may then run distally down the lumen 108 and out the open distal end of the sleeve body 106. In alternative examples, rather than being configured to receive irrigating fluid through the handpiece 86, the irrigation sleeve 104 may include a fitting in fluid communication with the irrigation conduit 124 and disposed on an outer surface of the sleeve body 106 for receiving the irrigation line 114 running outside of the handpiece 86. In this case, during operation of the ultrasonic instrument 18, irrigating fluid may be similarly flowed through the gap between the tip 82 and the sleeve body 106 via the fitting and out the open distal end of the sleeve body 106.

The ultrasonic instrument 18 may also define an aspiration pathway for providing suction at a surgical site through the distal region 84 of the tip 82. For instance, the tube 94 of the transducer 90 may define a lumen extending from the proximal end to the distal end of the transducer 90 to create a fluid passageway through the transducer 90. The horn 96 may similarly define a lumen extending from the proximal end to the distal end of the horn 96 to create a fluid passageway through the horn 96, and the tip 82 may also define a lumen extending from the proximal end to the distal end of the tip 82 to create a fluid passageway through the tip 82. Collectively, these lumens may form at least a portion of an aspiration pathway that extends from the distal region 84 of the tip 82 to the proximal end of the handpiece 86.

The ultrasonic instrument 18 may further include a fitting 128 coupled to the tube 94 and extending proximally from the proximal end of the handpiece 86 for receiving a suction line 130. During a procedure, suction may be applied to the fluid pathway defined by the tube 94, horn 96, and tip 82 via the fitting 128 and suction line 130 to draw the irrigating fluid applied to the surgical site and debris formed by a procedure that is entrained in the fluid towards and out of the proximal end of the handpiece 86. More specifically, the control console 80 may include a vacuum pump 132 in fluid communication with a waste canister 134 via the cassette 118 when inserted in the control console 80, with the waste canister 134 being separately placed in fluid communication with the fluid passageway defined by the tube 94, horn 96, and tip 82 via a fluid passageway defined by the fitting 128, suction line 130, and cassette 118. In this way, the vacuum pump 132 may apply a suction to the fluid passageway defined by the tube 94, horn 96, and tip 82 via the waste canister 134, fitting 128, suction line 130, and cassette 118, thereby drawing materials from the surgical site through the aforementioned fluid passageways into the waste canister 134.

In addition to aspirating the surgical site, the suction of surrounding air and dispensed irrigation fluid through the ultrasonic instrument 18 may assist in significantly reducing the temperature in the tip 82 and handpiece 86. The suction may also function to draw tissue towards the tip head 84, which may enhance the effectiveness of the tip 82 in sensing and treating patient tissue.

The control console 80 may also include a user interface 30B (FIG. 6) for receiving user input and providing information to the user. For instance, the control console 80 may include a display 136 for presenting information to the practitioner that forms a component of the user interface 30B. Non-limiting examples of presented information may include an identification of the ultrasonic instrument 18, or more particularly of the handpiece 86 and/or tip 82, currently connected to the control console 80, and an operating state of the ultrasonic system 12. The display 136 may be a touch screen display that enables the practitioner to provide input to the control console 80, such as via on-screen control elements.

An operator may interact with the on-screen control elements to set operational parameters of the ultrasonic system 12, such as an ultrasonic energy level, a suction level, an irrigation level, and/or a pulsing level for the ultrasonic instrument 18, such as described in PCT Publication No. WO 2022/072903 A1, the entire contents of which are hereby incorporated by reference herein. In some implementations, the control console 80 may be configured to determine a predefined maximum level for one or more of these operational parameters, such as based on data read from the ultrasonic instrument 18 when connected to the control console 80, and the settings for each such operational parameter input by the practitioner may indicate a percentage of the predefined maximum level to use for the ultrasonic instrument 18 relative to the operational parameter.

The user interface 30B of the ultrasonic system 12 may also include one or more actuation devices coupled to the control console 80. Upon activation by the practitioner, each of the actuation devices may cause the control console 80 to generate and source the AC drive signal to the ultrasonic instrument 18 that induces ultrasonic energy in the ultrasonic instrument 18 to vibrate the tip 82.

For instance, the one or more actuation devices may include a foot pedal 138. The foot pedal 138 may be wirelessly connected to the control console 80, such as via an adapter 140 connected to the control console 80. Upon being depressed, the foot pedal 138 may transition from an off position to an active position, and may communicate a corresponding signal to the control console 80 that indicates the depression. In some instances, the communicated signal may also vary with the extent to which the foot pedal 138 is depressed, such as to enable the practitioner to vary a target ultrasonic energy level (e.g., target power level) for the ultrasonic instrument 18 up to a maximum ultrasonic energy level for the ultrasonic instrument 18. The maximum ultrasonic energy level for the ultrasonic instrument 18 may be a predefined maximum level for the ultrasonic instrument 18, such as indicated by data read from the ultrasonic instrument 18, or to a practitioner-set maximum level, such as a percentage of the predefined maximum level as described above. Responsive to receiving the actuation signal, the control console 80 may generate and source an AC drive signal to the ultrasonic instrument 18 that causes the tip 82 to vibrate according to the current settings of the control console 80 and/or the extent of the depression indicated by the actuation signal.

The user interface 30B of the ultrasonic system 12 may also include a remote control 142 coupled to the control console 80. Similar to the display 136, the remote control 142 may provide practitioner-selectable elements for enabling practitioner input to the control console 80. For instance, the remote control 142 may include buttons for setting the operational parameters of the ultrasonic system 12, such as the ultrasonic energy level, suction level, irrigation level, and/or pulsing level for the ultrasonic instrument 18. The remote control 142 may also include a main power switch for turning on and off the control console 80. Additionally, or alternatively, the control console 80 may include a main power switch 144 for turning on and off the control console 80.

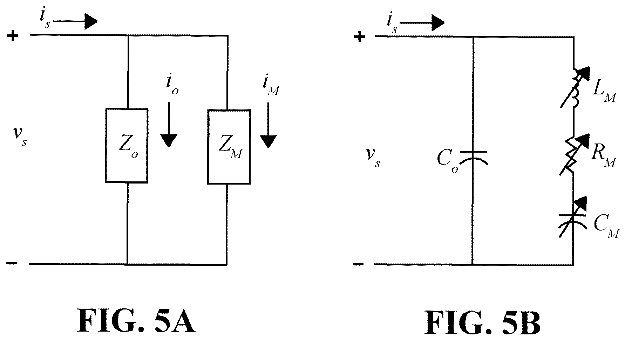

FIGS. 5A and 5B show circuits corresponding to an electro-mechanical approximation model that may represent both the mechanical and electrical properties of the ultrasonic instrument 18 when operating near resonance in an analogous electrical circuit. As shown in the illustrated examples, the current $i_s$ of the AC drive signal sourced to the ultrasonic instrument 18 may be broken down into two components: a current $i_o$ applied to the drivers 92 of the ultrasonic instrument 18 and an equivalent of current $i_M$ applied to the mechanical components of the ultrasonic instrument 18 (also referred to herein as "mechanical current $i_M$"). The mechanical components of the ultrasonic instrument 18 may include those components that vibrate in response to the sourced AC drive signal to treat patient tissue, such as and without limitation, the drivers 92, tube 94, horn 96, tip 82, and proximal end mass 95 described above.

The impedance $Z_o$ provided by the drivers 92 may be primarily capacitive. Accordingly, the drivers 92 may be represented by a capacitor with capacitance $C_o$. The capacitance $C_o$ of the drivers 92 may remain substantially constant during operation of the ultrasonic instrument 18, and may thus be determined and provided to the control console 80 in advance of an operation, such as upon connection of the ultrasonic instrument 18 to the control console 80, so as to tailor operation of the control console 80 to the specific handpiece 86 of the ultrasonic instrument 18. Additionally, or alternatively, the control console 80 may be configured to periodically measure the capacitance $C_O$ of the drivers 26 during operation of the ultrasonic instrument 18 to enable even further precision. For instance, the capacitance $C_O$ of the drivers 92 can change due to aging, because of temperature changes of the drivers 92, and/or relaxation of the crystalline structure after being at elevated temperatures.

The equivalent of impedance $Z_M$ provided by the mechanical components of the ultrasonic instrument 18 (also referred to herein as "mechanical impedance $Z_M$") may include an inductive component, a resistive component, and a capacitive component. Accordingly, the mechanical components may be represented by an inductor with inductance $L_M$, a resistor with resistance $R_M$, and a capacitor with capacitance $C_M$. The mechanical impedance $Z_M$ may vary with operation of the ultrasonic instrument 18. For instance, at least the resistance $R_M$ (also referred to herein as "mechanical resistance $R_M$") may vary as a function of mechanical damping imposed by tissue contacted by the distal region 84 of the tip 82 and/or irrigation fluid in contact with the tip, and at least the capacitance $C_M$ may vary as a function of the mechanical stiffness of tissue contacted by the distal region 84 of the tip 82 and/or by the temperature of the tip 82, such as due to changes in the speed of sound and stiffness of the material of the tip 82 as a function of the temperature of the tip 82. In some implementations, the mechanical impedance $Z_M$ may therefore be used to determine whether the distal region 84 of the tip 82 is loaded with tissue, and/or to determine a type of the loaded tissue, as described in more detail below.

The ultrasonic energy induced in the ultrasonic instrument 18, and correspondingly the vibrations of the tip 82, may be proportional to the mechanical current $i_M$ induced in the ultrasonic instrument 18. More specifically, the frequency of the vibrations at the tip head 84 may be related (e.g., equal) to the frequency of the mechanical current $i_M$. The amplitude of the vibrations of the tip head 84 may be related to (e.g., an integral of) the velocity of the tip head 84, which in turn may be related to (e.g., proportional to) the amplitude of the mechanical current $i_M$. For instance, when the ultrasonic instrument 18 is operating at resonance, the peak-to-peak displacement of the tip head 84 in microns may be approximately 180% to 220% of the amplitude of the mechanical current $i_M$ in milliamps, depending on the gain of the tip 82. As an example, a mechanical current $i_M$ at the resonant frequency of the ultrasonic instrument 18 and with an amplitude of 150 milliamps may induce the tip head 84 of a given tip 82 to vibrate back and forth along a path of travel that is approximately 330 microns. The control console 80 may thus cause vibrations in the tip 82 with a target frequency and displacement level by sourcing an AC drive signal to the ultrasonic instrument 18 that induces a mechanical current $i_M$ in the ultrasonic instrument 18 with the target frequency and an amplitude corresponding to the target displacement level.

Referring again to FIG. 6, the control console 80 may include an ultrasonic controller 150, console storage 152, and a power supply 154 for generating the AC drive signal sourced to the ultrasonic instrument 18, such as according to parameters set by the ultrasonic controller 150. Specifically, the ultrasonic controller 150 may be configured to assert control signals to the power supply 154 that regulate the AC drive signal output to the ultrasonic instrument 18.

The power supply 154 may include a DC supply 156, an amplifier 158, and a transformer 160. During operation of the ultrasonic system 12, the DC supply 156 may output a DC signal to a center tap of a primary winding 162 of the transformer 160. The opposed ends of the transformer primary winding 162 may be tied to the amplifier 158, which may be configured to develop an AC signal across the primary winding 162 from the applied DC signal by applying varying levels of resistance to the ends of the transformer primary winding 162. The AC signal developed across the primary winding 162 may induce a proportional AC drive signal across a secondary winding 164 of the transformer 160, which may be coupled to the ultrasonic instrument 18 through electrical contacts 166. A further understanding of the assemblies of the power supply 154 can be found in PCT Publication No. WO 2016/183084 A1, the contents of which are hereby incorporated by reference herein in their entirety.

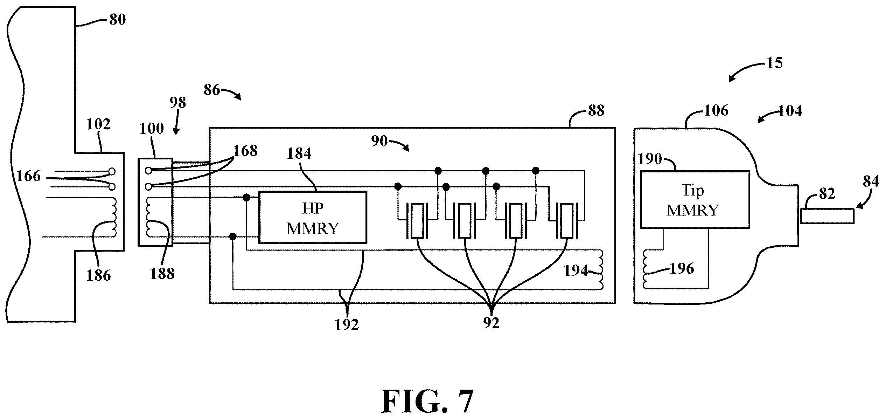

As illustrated in FIG. 7, the electrical contacts 166 may be integral with the socket 102 of the control console 80. Corresponding electrical contacts 168 may be integral with the adapter 100 of the electrical cable 98. When the adapter 100 is fully seated in the socket 102, the electrical contacts 166, 168 may become aligned and form an electrical path over which the AC drive signal across the secondary winding 164 of the transformer 160 is sourced to the ultrasonic instrument 18 to vibrate the tip 82. The drivers 92 may be disposed within the handpiece 86 so that the AC drive signal is applied to each of the drivers 92 in parallel, and thereby causes vibrations of the tip 82 according to the received AC drive signal.

Referring again to FIG. 6, the ultrasonic controller 150 may be configured to implement the functions, features, processes, and methods of the control console 80 described herein. For instance, the ultrasonic controller 150 may be configured to determine whether to operate the ultrasonic instrument 18 in a tissue sense mode or a tissue resection mode, and to regulate the AC drive signal developed across the secondary winding 164 accordingly. The ultrasonic controller 150 may be configured to regulate the AC drive signal developed across the secondary winding 164 by regulating the AC signal developed across the primary winding 162, such as by supplying a control signal to the DC supply 156 that sets the potential of the signal applied to the center tap of the primary winding 162, and/or by supplying a control signal to the amplifier 158 that corresponds to a target AC drive signal to be developed across the secondary winding 164.

For instance, the ultrasonic controller 150 may be configured to induce a target AC drive signal across the secondary winding 164 by supplying a control signal to the amplifier 158 that includes a frequency and amplitude corresponding to that of the target AC drive signal. In some implementations, the ultrasonic controller 150 may be configured with a direct digital synthesis (DDS) component for generating the control signal. Responsive to receiving the control signal, the amplifier 158 may be configured to develop an AC signal across the primary winding 162 that is proportional to and may induce the target AC drive signal across the secondary winding 164 for being sourced to the ultrasonic instrument 18. By regulating the AC signals developed across the primary winding 162 and secondary winding 164 of the transformer 160 in this manner, the ultrasonic controller 150 may be configured to control both the frequency, velocity, and amplitude of the vibrations of the tip 82.

In some implementations, the ultrasonic controller 150 may be configured to source a pulsed AC drive signal to the ultrasonic instrument 18, such that the amplitude of the AC drive signal varies between an upper amplitude and a lower amplitude as a function of time. To this end, the ultrasonic controller 150 may be configured to generate, such as using DDS, a base AC signal with an ultrasonic frequency and a constant amplitude, and a modulation signal for amplitude modulating the base AC signal. The ultrasonic controller 150 may then be configured to combine the signals, such as using a multiplier, to generate a control signal for the power supply 154 that corresponds to the target pulsed AC drive signal.

The ultrasonic controller 150 may be configured to determine whether to initiate vibration of the tip 82 of the ultrasonic instrument 18 by monitoring the state of the foot pedal 138. Responsive to the foot pedal 138 being depressed, the ultrasonic controller 150 may be configured to cause the power supply 154, such as via a corresponding control signal from the ultrasonic controller 150, to source an AC drive signal to the ultrasonic instrument 18 that corresponds to a target ultrasonic energy level for the ultrasonic instrument 18, such as determined as a function of the extent of depression of the foot pedal 138, settings of the control console 80, a predefined maximum ultrasonic energy level for the ultrasonic instrument 18, and/or an operational mode of the ultrasonic system 12 as described herein.

As previously described, the ultrasonic controller 150 may be configured to cause vibrations in the tip head 84 with a target frequency and displacement level by generating a control signal to the power supply 154 that causes the power supply 154 to source an AC drive signal to the ultrasonic instrument 18 that induces a mechanical current $i_M$ in the ultrasonic instrument 18 with the target frequency and an amplitude corresponding to the target displacement level. To this end, the ultrasonic controller 150 may be configured to implement two control loops to induce target vibrations in the tip 82, namely, a control loop for regulating the frequency of the mechanical current $i_M$ induced in the ultrasonic instrument 18 by the AC drive signal, and a control loop for regulating the level or amplitude of the mechanical current $i_M$ induced in the ultrasonic instrument 18 by the AC drive signal. Each control loop may incorporate a PID controller for efficiently adjusting the AC drive signal to achieve the desired values, and may have an iterative loop time of approximately 400 microseconds.

Referring now to FIGS. 4A, 4B, and 6, using Ohm's law, the ultrasonic controller 150 may be configured to calculate the level of mechanical current $i_M$ induced in the ultrasonic instrument 18 by a given AC drive signal using the following Equation:

$$i_M = i_S - j2\pi f C_o v_s \qquad (1)$$

where $i_S$ is the current of the AC drive signal being sourced to the ultrasonic instrument 18, $f$ is the frequency of the AC drive signal being sourced to the ultrasonic instrument 18, $C_o$ is the capacitance of the drivers 92, and $v_s$ is the voltage of the AC drive signal being sourced to the ultrasonic instrument 18. An explanation for Equation (1) is provided in Applicant's U.S. Patent No. 10,016,209, the contents of which are hereby incorporated by reference herein in their entirety. Assuming the frequency $f$ of the AC drive signal has been previously set to acheive a desired vibratory characteristic of the ultrasonic instrument 18 (e.g., resonance), the ultrasonic controller 150 may induce a target level of mechanical current $i_M$, and correspondingly target vibrations of the tip 82, by setting the voltage $v_s$ of the AC drive signal so that Equation (1) results in the target level of mechanical current $i_M$.

As mentioned above, a characteristic integral with the ultrasonic instrument 18 is the mechanical resonant frequency of the ultrasonic instrument 18. The mechanical resonant frequency is a frequency at which the distal region 84 of the tip 82 undergoes vibratory motions of a peak range. In other words, assuming other electrical characteristics remain constant, at the resonant frequency, the tip 82 undergoes a motion that is larger in magnitude than a motion that would occur if the drivers 92 were vibrated at a frequency less than or greater than the resonant frequency. For a tip 82 that vibrates longitudinally, the peak range may be understood as the largest back and forth distance of the distal region 84 of the tip 82.

Applicant's U.S. Patent No. 10,016,209 also discloses a means for tracking the resonant frequency of the ultrasonic instrument 18, which may vary during operation of the ultrasonic instrument 18. In particular, the ultrasonic instrument 18 may be considered as operating at resonance when the real part of the ratio of the current $i_o$ through the drivers 92 to the mechanical current $i_M$ is substantially equal to zero. The ultrasonic controller 150 may thus be configured to determine the resonant frequency of the ultrasonic instrument 18 by determining a value for the frequency $f$ of the AC drive signal such that the following Equation is true:

$$Re\left\{\frac{j2\pi f C_o}{i_s - j2\pi f C_o}\right\} \approx 0 \qquad\qquad (2)$$

where $i_s$ is the current of the AC drive signal sourced to the ultrasonic instrument 18 and $C_o$ is the capacitance of the drivers 92. Responsive to determining the resonant frequency of the ultrasonic instrument 18, such as using Equation (2), the ultrasonic controller 150 may be configured to set the frequency of the AC drive signal to the determined resonant frequency, thereby causing the ultrasonic instrument 18 to operate at resonance.

As the frequency of the AC drive signal is adjusted to follow a target vibratory characteristic of the ultrasonic instrument 18 such as resonance, the level of the mechanical current $i_M$ induced in the ultrasonic instrument 18 may vary. Accordingly, to induce target ultrasonic energy in the ultrasonic instrument 18, the ultrasonic controller 150 may be configured to repeatedly alternate between or perform in parallel the operations of regulating the frequency of the AC drive signal based on Equation (2) and setting the voltage $v_s$ of the AC drive signal so that the mechanical current $i_M$, calculated according to Equation (1), corresponds to the target ultrasonic energy level.

To this end, the ultrasonic controller 150 may be configured to receive feedback data corresponding to the AC drive signal sourced to the ultrasonic instrument 18, such as via one or more sensors of the control console 80. For example, referring to FIG. 6, the control console 80 may include a sensor for measuring a voltage $v_s$ of the AC drive signal sourced to the ultrasonic instrument 18, which may include a tickler coil 174 integral with the transformer 160. The ticker coil 174 may be connected to a voltage measuring circuit 176 of the control console 80, which in turn may be connected to the ultrasonic controller 150. The signal across ticker coil 174 may have a known relationship to the voltage $v_s$ of the AC drive signal being sourced to the ultrasonic instrument 18. Based on the signal across the ticker coil 174, the voltage measuring circuit 176 may generate and communicate a signal to the ultrasonic controller 150 representative of the potential and phase of the voltage $v_s$ of the AC drive signal being applied to the ultrasonic instrument 18. The ultrasonic controller 150 may thus be configured to measure the voltage $v_s$ of the sourced AC drive signal via the voltage measuring circuit 176 and ticker coil 174, and to take actions based thereon.

As a further example, the control console 80 may include a sensor for measuring a current $i_s$ of the AC drive signal being sourced to the ultrasonic instrument 18, which may include a coil 178 located in close proximity to one of the conductors that extends from the secondary winding 164 of the transformer 160 to the ultrasonic instrument 18. The coil 178 may be connected to a current measuring circuit 180 of the control console 80, which in turn may be connected to the ultrasonic controller 150. The signal across the coil 178 may have a known relationship to the current $i_s$ of the AC drive signal being sourced to the ultrasonic instrument 18. Based on the signal across coil 178, the current measuring circuit 180 may produce and communicate to the ultrasonic controller 150 a signal representative of the magnitude and phase of the current $i_s$ of the AC drive signal being applied to the ultrasonic instrument 18. The ultrasonic controller 150 may thus be configured to measure the current $i_s$ of the sourced AC drive signal via the current measuring circuit 180 and coil 178, and to take action based thereon.

As described above, the ultrasonic controller 150 may be configured to regulate the frequency and voltage $v_s$ of the sourced AC drive signal based on the obtained measurements of the voltage $v_s$ and/or current $i_s$ of the AC drive signal. Further during operation of the ultrasonic instrument 18, the control console 80, or more particularly the ultrasonic controller 150, may be configured to determine whether the ultrasonic instrument 18 is in a loaded state (e.g., contacting patient tissue) based on obtained electrical characteristics of the sourced AC drive signal, such as measurements of the voltage $v_s$ and/or the current $i_s$ of the AC drive signal, and if so, determine the type of the contacted patient tissue based on electrical characteristics of the AC drive signal, such as a set frequency $f$ the AC drive signal, which may have been previously determined to correspond to the resonant frequency of the ultrasonic instrument 18 as described above.

For instance, during operation of the ultrasonic instrument 18, the ultrasonic controller 150 may be configured to calculate the mechanical impedance $Z_M$ of the ultrasonic instrument 18 by dividing the mechanical current $i_M$ of the ultrasonic instrument 18, such as calculated as described above, by the measured voltage $v_s$ of the AC drive signal. Assuming the ultrasonic instrument 18 is operating at resonance, the inductive component $L_M$ of the mechanical impedance $Z_M$ may generally cancel out the capacitive component $C_M$ of the mechanical impedance $Z_M$, in which case the mechanical impedance $Z_M$ may generally equal the mechanical resistance $R_M$. To determine whether the ultrasonic instrument 18 is in a loaded state, the ultrasonic controller 150 may be configured to compare the calculated mechanical impedance $Z_M$, or more particularly the mechanical resistance $R_M$, to a threshold corresponding to the ultrasonic instrument 18 being in a loaded state. In some implementations, the threshold may be specific to the ultrasonic handpiece 18 and/or tip 82 coupled to the console 80, and/or may be read from the ultrasonic instrument 18 as described below. Responsive to the calculated mechanical impedance $Z_M$ exceeding the threshold, the ultrasonic controller 150 may be configured to determine that the ultrasonic instrument 18 is in a loaded state.

Responsive to determining a loaded state, the ultrasonic controller 150 may be configured to utilize the resonant frequency and/or mechanical impedance $Z_M$, or more particularly the mechanical resistance $R_M$, of the ultrasonic instrument 18 calculated from the obtained measurements as described above to determine a type of the contacted tissue. The ultrasonic controller 150, such as in cooperation with the navigation controller 26 and/or robotic controller 64, may then be configured to adjust operational parameters of the surgical system 10 based on the determined tissue type.

For instance, responsive to determining contact with a given type of tissue, the ultrasonic controller 150 may be configured to induce ultrasonic energy in the ultrasonic instrument 18 according to a predefined ultrasonic energy profile associated with the determined tissue type within the console storage 152, which may define a specific frequency, mechanical current $i_M$, and/or pulsing profile for the AC drive signal sourced to the ultrasonic instrument 18 to resect the given type of tissue.

In one example, the ultrasonic controller 150 may be configured to initially operate the ultrasonic instrument 18 in a tissue sense mode. In the tissue sense mode, the ultrasonic controller 150 may induce ultrasonic energy in the ultrasonic instrument 18 that is sufficient for tracking a resonant frequency and/or mechanical impedance $Z_M$ of the ultrasonic instrument 18, but not resection of tissue being contacted by the distal region 84 of the tip 82. Responsive to determining contact with a tissue type corresponding to tissue targeted for resection, the ultrasonic controller 150 may be configured to transition operation of the ultrasonic instrument 18 from the tissue sense mode to a tissue resection mode to cause resection of the contacted tissue. In the tissue resection mode, the ultrasonic controller 150 may be configured to induce ultrasonic energy in the ultrasonic instrument 18 of a higher level sufficient to resect contacted patient tissue.

The ultrasonic controller 150 may also be configured to sense a type of patient tissue being contacted by the distal region 84 of the ultrasonic instrument 18 during operation of the ultrasonic instrument 18 in the tissue resection mode, such as based on determined electrical characteristics of the AC drive signal (e.g., measurements of the voltage $v_s$ and/or current $i_s$ of the sourced AC drive signal, a set frequency $f$ of the AC drive signal). Tissue type may refer to an identification of the tissue, and/or may refer to characteristics of the tissue, such as the relative stiffness or density of the tissue. The ultrasonic controller 150 may be configured to display the sensed tissue type throughout the procedure. Responsive to determining contact by the ultrasonic instrument 18 with a type of tissue corresponding to non-targeted tissue when operating in the tissue resection mode, the ultrasonic controller 150 may be configured to transition operation of the ultrasonic instrument 18 from the tissue resection mode back to the tissue sense mode. Additionally or alternatively, responsive to detecting a tissue type corresponding to tissue that is targeted for resection but located near a critical structure or other non-targeted object, such as when the ultrasonic instrument 18 is being used to resect bone and is determined to be near breaking through an outer wall of the bone based on the detected tissue type, the ultrasonic controller 150 may be configured to cease operation of the ultrasonic instrument 18, or to continue operation of the ultrasonic instrument 18 in the tissue resection mode but at a reduced power level, such as by reducing the target ultrasonic energy level and/or pulsing the AC drive signal so as to induce vibratory pulses extending between a set target ultrasonic energy level and a lower level, for instance as disclosed in PCT Application No. PCT/US2023/017790.

Additionally, or alternatively, the navigation controller 26 may be configured to adjust the virtual objects associated with the tracked objects of interest, which may include varying types of tissue, based on the determined type of tissue. For instance, responsive to a determination of contact by the ultrasonic instrument 18 with a given tissue type, the navigation controller 26, in cooperation with the ultrasonic controller 150 of the ultrasonic system 12, may be configured to determine whether the determined tissue type corresponds to that indicated by the tracked position of the distal region 84 of the tip 82 relative to a virtual object associated with the determined tissue type. If not, then the navigation controller 26 may be configured to adjust a parameter (e.g., shape, position, pose, force/torque control parameter) of the virtual object in the known coordinate system so that the tracked position of the distal region 84 of the tip 82 relative to the virtual object likewise indicates contact by the distal region 84 of the tip 82 with the determined tissue type.

As a further example, responsive to the determined tissue type corresponding an anatomical structure of interest (e.g., bone), the navigation controller 26 may be configured to automatically begin tracing the patient anatomy by tracking the movement of the distal region 84 of the tip 82, such as so long as the determined tissue type continues to correspond to the anatomical structure of interest, so as generate a virtual model of the anatomical structure. For example: when performing a discectomy, each time the ultrasonic controller 150 senses contact with a bony structure, the navigation controller 26 may be configured to store a point corresponding to the current position of the distal region 84 of the tip 82 indicated by the tracking data. After gathering multiple points, this point cloud can be fitted to the surface of the closest vertebra to update the virtual model and/or virtual objects associated with the vertebra in the known coordinate system, which in turn may account for vertebral motion.

Additionally or alternatively, the navigation controller 26 may be configured to cooperate with the ultrasonic controller 150 to track a resection status of a surgical procedure based on the determined tissue types indicated by the electrical characteristics of the AC drive signal and the tracked poses of the ultrasonic instrument 18 corresponding in time with the determined tissue types. More specifically, based on the tissue types determined with the ultrasonic instrument 18 from the electrical characteristics of the AC drive signal, the navigation controller 26 may be configured to calculate an estimated volume of tissue targeted for resection that has been resected with the ultrasonic instrument 18, and compare the calculated volume to a planned resection volume, such as determined based on preoperative medical images of the target site. The navigation controller 26 may then be configured to display the resection status via the user interface 30A, such as in the form of a percentage, or a graphic showing a virtual model or medical image corresponding to the target site with highlighting indicative of portions of the target volume that been resected and/or portions not yet resected.

The above sensing and other functionality of the ultrasonic controller 150 and/or navigation controller 26 may be implemented as described as in Applicant's U.S. Provisional Patent Application 63/589,988, filed October 12, 2023 and titled "Tissue Sensing with an Ultrasonic Resection Tool", the entire contents of which are hereby incorporated by reference herein.

Still referring to FIG. 6, the console storage 152 may store data supporting the functions, features, processes, and methods of the control console 80, or more particularly of the ultrasonic controller 150, described herein. For example, and without limitation, the console storage 152 may store waveform data 170 and tissue type data 172. The waveform data 170 may include one or more DDS arrays each representative of a different waveform, such as a sine waveform, and may be utilized by the ultrasonic controller 150 to generate control signals for the power supply 154 as described above.

The tissue type data 172 may map varying potential electrical characteristics of an ultrasonic instrument 18, or more particularly of the sourced AC drive signal, such as varying resonant frequencies and/or mechanical impedances $Z_M$ calculated from measured characteristics of the AC drive signal, with varying types of tissue. In some implementations, the associations between the electrical characteristics and tissue types indicated by the tissue type data 172 may also be a function of force being applied to the ultrasonic instrument 18, such as according to that indicated by the force-torque sensors 66, and/or of the type of handpiece 86 and/or tip 82 being used. In other words, the same or similar electrical characteristics may correspond to different types of patient tissue as a function of varying forces and/or varying handpiece 86 and/or tip 82 combinations. Thus, responsive to determining one or more characteristics of the ultrasonic instrument 18 related to contacted tissue type such as described above, the ultrasonic controller 150 may be configured determine the type of tissue being contacted with the ultrasonic instrument 18 by querying such characteristics against the tissue type data 172.

The control console 80 may also include a memory reader 182 for communicating with one or more electronic memory storage devices integral with the ultrasonic instrument 18. The ultrasonic instrument 18 may include one or more electronic memory storage devices for storing data that identifies the ultrasonic instrument 18, or more particularly the handpiece 86 and/or tip 82. The stored data may also define operational data specific to the ultrasonic instrument 18, or more particularly the handpiece 86 and/or tip 82. Non-limiting examples of operational data may include a maximum current for the sourced AC drive signal, a maximum mechanical current $i_M$ for the sourced AC drive signal, a maximum voltage for the sourced AC drive signal, minimum and maximum frequencies for the sourced AC drive signal, PID coefficients for regulating the sourced AC drive signal, a capacitance of the drivers 92, and a use history.

In some implementations, the operational data may additionally or alternatively include data indicating whether the ultrasonic instrument 18, or more particularly the handpiece 86 and/or tip 82, is enabled for tissue sense mode and/or tissue resection mode with a sensing function, and data specific to the ultrasonic instrument 18 for facilitating such modes as described herein.

In some implementations, the stored data may also define calibration data specific to the ultrasonic instrument 18, or more particularly the handpiece 86 and/or tip 82. The calibration data may generally indicate values for normalizing determined electrical characteristics of an ultrasonic instrument 18 to the specific attributes of the ultrasonic instrument 18, or more particularly of the specific handpiece 86 and/or tip 82, for determining a type of tissue being contacted by the ultrasonic instrument 18, and/or forces being applied on the ultrasonic instrument 18 by the surgeon and/or the robotic manipulator 16 in one or more degrees of freedom. For instance, the calibration data may indicate a conversion factor for converting electrical characteristics of the ultrasonic instrument 18 to mechanical characteristics of the ultrasonic instrument 18, and/or vice versa. The calibration data may also indicate the gain of the tip 82 (e.g., distal end displacement divided by proximal horn displacement). The conversion factor and/or gain may be used by the ultrasonic controller 150 to determine an impedance of the contacted tissue from the determined electrical characteristics, which in turn may be used to infer or represent the type of tissue being contacted by the ultrasonic instrument 18.

For instance, referring again to FIG. 7, the handpiece 86 of the ultrasonic instrument 18 may include a handpiece (HP) memory 184 disposed therein. As non-limiting examples, the HP memory 184 may be an EPROM, an EEPROM, or an RFID tag. Responsive to connecting the ultrasonic instrument 18 to the control console 80, the ultrasonic controller 150 may be configured to read the data stored in the HP memory 184 using the memory reader 182, and to tailor operation of the control console 80 based on the read data. More particularly, the control console 80 may include a communication interface, such as a coil 186, connected to the memory reader 182. The coil 186 may be integral with the socket 102 of the control console 80. The HP memory 184 may similarly be connected to a coil 188, which may be integral with the adapter 100 of the cable 98. When the ultrasonic instrument 18 is connected to the control console 80 via the power cable 98, the coils 186, 188 may become aligned and able to inductively exchange signals. The ultrasonic controller 150 may then be configured to read data from and write data to the HP memory 184 over the coils 186, 188.

More particularly, the memory reader 182 may be configured to convert signals across the coil 186 into data signals readable by the ultrasonic controller 150. The memory reader 182 may also be configured to receive data to be written to the HP memory 184 from the ultrasonic controller 150, and to generate a signal across the coil 186 that causes the data to be written to the HP memory 184. The structure of the memory reader 182 may complement that of the HP memory 184. Thus, continuing with the above non-limiting examples, the memory reader 182 may be an assembly capable of reading data from and writing data to an EPROM, EEPROM, or RFID tag.

In addition, or alternatively to the HP memory 184, the ultrasonic instrument 18 may include a tip memory 190. As described above, the tip 82 may be removable from the handpiece 86 so the handpiece 86 can be used with varying interchangeable tips 82, and different tips 82 may have different structural characteristics and operational limitations. Accordingly, the HP memory 184 may store data identifying the handpiece 86 and operational parameters specific to the handpiece 86, including the capacitance of the drivers 92 and operational parameters specific to the secondary drive component of the sourced AC drive signal (which may be powered through the handpiece 86), and the tip memory 190 may store data identifying the tip 82 currently coupled to the handpiece 86 and operational parameters specific to the tip 82. The HP memory 184 may similarly store calibration data specific to the ultrasonic handpiece 86, such as the above-described conversion factor, and the tip memory 190 store calibration data specific to the tip 82, such as the gain of the tip 82.

Because the tip 82 and sleeve 104 may be distributed together as a single package, the tip memory 190 may be disposed in the sleeve 104. In one example, the tip memory 190 may also store data corresponding to the previously described target data 56 and/or tissue type data 172 that is specific to the tip 82. The tip memory 190 may be the same type of memory as the HP memory 184 (e.g., an EPROM, an EEPROM, or an RFID tag).

Responsive to connecting the ultrasonic instrument 18 to the control console 80, the ultrasonic controller 150 may be configured to read the data stored in the HP memory 184 and the tip memory 190 using the memory reader 182, and to tailor operation of the control console 80 to the specific handpiece 86 and tip 82 combination coupled to the control console 80. In some instances, the tip memory 190 may include values for the same operational and/or calibration parameters as the HP memory 184. To the extent the values for a given parameter differ between the HP memory 184 and the tip memory 190, the ultrasonic controller 150 may be configured to utilize the more restrictive value to manage operation of the ultrasonic instrument 18. Additionally or alternatively, to the extent the both the HP memory 184 and the tip memory 190 include a value for a given parameter, the ultrasonic controller 150 may be configured to derive a value (e.g., max current for the main drive component of the AC drive signal) to manage operation of the ultrasonic instrument 18 based on a combination of the values stored in the memories (e.g., summing or averaging the values).

Similar to the HP memory 184, the ultrasonic controller 150 may read data from and write data to the tip memory 190 via the memory reader 182 and coil 186. In particular, the handpiece 86 may include two conductors 192 extending from the proximal region to the distal region of the handpiece 86. The proximal ends of the conductors 192 may be coupled to the coil 188, which may be integral with the adapter 100 of the electrical cable 98. The distal ends of the conductors 192 may be coupled to another coil 194 disposed at the distal region of the handpiece 86. A corresponding coil 196 may be disposed in a proximal region of the sleeve 104. When the sleeve 104 is disposed around the tip 82 and fitted to the handpiece 86, the coils 194, 196 may become aligned and able to inductively exchange signals. When the handpiece 86 is connected to the control console 80 via the cable 98, the coils 186, 188 may also become aligned and able to inductively exchange signals. The ultrasonic controller 150 may then read data from and write data to the tip memory 190 over the conductors 192 via inductive communication provided by the coils 186, 188 and the coils 194, 196.

Referring again to FIG. 6, the ultrasonic controller 150 may also be coupled and configured to drive the display 136 of the control console 80, and may be coupled to the remote control 142. As previously described, the ultrasonic controller 150 may be configured to generate information and user interface (UI) components for presentation on the display 136, and when the display 136 is a touch screen display, the ultrasonic controller 150 may also be configured to cause the display 136 to depict images of buttons and other user-selectable components for setting desired operating parameters for the ultrasonic system 12. The remote control 142 may likewise include user-interactable elements for setting desired operating parameters of the ultrasonic system 12 from a position remote from the control console 80.

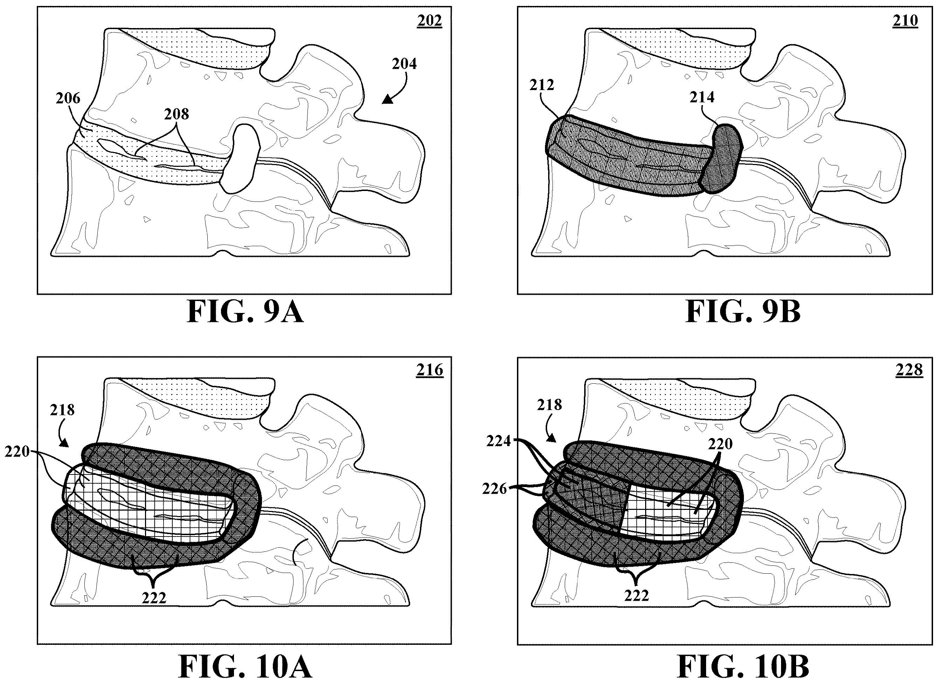

At least two of the controllers 26, 64, 150 of the surgical system 10 may form a control system for implementing various methods of operating the robotic manipulator 16 to control movement of the ultrasonic instrument 18 to a target site for resecting the target site. In one exemplary method, prior to a surgical procedure, a medical image of patient tissue including at least one target site may be received by the control system, such as by the navigation controller 26, and may be displayed via the user interface 30A. For instance, FIG. 9A illustrates a screen 202 that may be generated by the control system, such as the navigation controller 26, that shows a medical image of a section 204 of a patient's spine. As shown in the illustrated example, the section 204 includes an intervertebral disc space 206 including tears 208, which may define a target site with tissue targeted for resection during a surgical procedure.

As illustrated in the exemplary screen 210 of FIG. 9B, following manual and/or automated segmentation of the medical image by the control system, such as the navigation controller 26, the control system, such as the navigation controller 26, may be configured to display one or more regions of interest relative to the medical image. For instance, the screen 210 may illustrate a region 212 corresponding to tissue targeted for resection and/or a region 214 corresponding to an area to be avoided. Each of the regions of interest may correspond to an object in the surgical workspace to be tracked in the known coordinate system, such as a target site and non-targeted tissue adjacent the target site.

As described above, the control system, such as the navigation controller 26, may be configured to determine a pose of a coordinate system of the medical image in a known coordinate system in which a pose of the ultrasonic instrument 18 is also tracked. For instance, the control system may be configured to track the pose of an imaging system tracker 20 coupled to the imaging device in the known coordinate system, and reference the relationship data 50 defining a predetermined relationship between the imaging system tracker 20 and the coordinate system of the medical image to determine a pose of the coordinate system of the medical image in the known coordinate system. Alternatively, the control system, such as the navigation controller 26, may be configured to track the position of the distal region 84 of the tip 82 of the ultrasonic instrument 18 or the distal region of the calibration tool 52 in the known coordinate system as it is touched off various points represented in the medical image, with the user contemporaneously interacting with the user interface 30A to indicate a position within the medical image that correlates with each tracked position.

The control system, such as the navigation controller 26, may be configured to determine the poses of patient tissues of interest that are represented in the medical image in the known coordinate system based on the determined pose of the image coordinate system in the known coordinate system and/or patient trackers 20A disposed relative to such tissue as described above. The control system, such as the navigation controller 26, may also be configured to generate a tissue-density map for the patient tissues of interest in the known coordinate system based on the medical image and the determined pose of the image coordinate system in the known coordinate system. The tissue-density map may indicate varying densities of the patient tissues of interest as represented by the medical image relative to the known coordinate system.

The control system may use such information to confirm the type of patient tissue being contacted by the distal region 84 of the tip 82 of the ultrasonic instrument 18 and/or identify a force being applied on the ultrasonic instrument 18, such as by the surgeon and/or robotic manipulator 16, towards the contacted patient tissue. For instance, the tissue type data 172 may associate varying potential electrical characteristics of the ultrasonic instrument 18, or more particularly of the AC drive signal sourced to the ultrasonic instrument 19, with varying tissue densities. Responsive to determining one or more electrical characteristics of the ultrasonic instrument 18 as described above, the control system, such as the ultrasonic controller 150, may query such tissue type data 172 to verify that the tissue density associated with the determined electrical characteristic(s) generally corresponds to the tissue density indicated by the tissue-density map at the tracked position of the distal region 84 of the tip 82 in the known coordinate system, such as determined by the navigation controller 26. If not, then the control system, such as the navigation controller 26, may be configured to halt operation of the ultrasonic instrument 18, and/or instruct the robotic manipulator 16 to halt movement or retract the ultrasonic instrument 18 away from the target site. With a sufficient number of data points each indicating a tissue density indicated by the electrical characteristics of the ultrasonic instrument 18 and a tissue density indicated by the tissue-density map at a position according to corresponding tracking data, the control system, such as the navigation controller 26, may be configured to adjust the tissue-density map and/or virtual objects associated with patient tissues in the known coordinate system so that the tissue density indicated by the tracking data of each mismatched data point matches the tissue density indicated by the electrical characteristics of the data point.

Additionally or alternatively, the tissue type data 172 may indicate a force being applied to the ultrasonic instrument 18 relative to contacted tissue as a function of one or more determined electrical characteristics of the ultrasonic instrument 18, or more particularly of the sourced AC drive signal (e.g., the voltage $v_s$ of the AC drive signal), and the tissue density of the contacted tissue, such as indicated by the tissue-density map at a position indicated by the tracking data. Thus, responsive to the tracking data indicating that the distal region 84 of the tip 82 is contacting a given type of tissue, or more particularly a tissue of a given density, the control system, such as the ultrasonic controller 150, may be configured to query the tissue type data 172 to infer an amount of force being applied on the ultrasonic instrument 18 relative to the contacted tissue based on determined electrical characteristic(s) of the AC drive signal and the tissue density indicated by the tracking data. Such force may then be used to control the robotic manipulator 16 to provide a target force, as described herein.

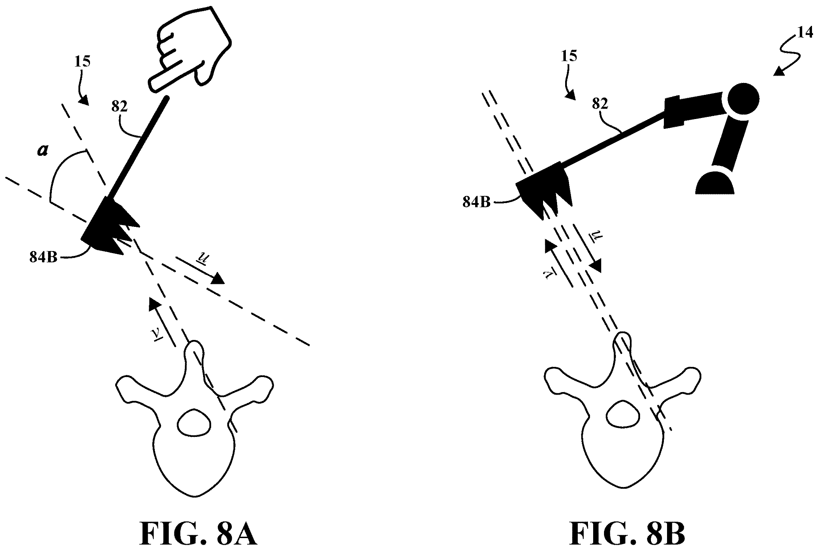

One or more target parameters for controlling the robotic manipulator 16 to move and/or guide movement the ultrasonic instrument 18 may also be determined prior to the start of the surgical procedure. As previously described, the ultrasonic instrument 18 may be used with interchangeable tips 82 with varying distal regions 84, each having a different sweet spot relative to force applied on the ultrasonic instrument 18 for sensing and/or resecting patient tissue using the tip 82, and/or relative to a pose of the distal region 84 of the tip 82 for sensing and/or resecting patient tissue using the tip 82. For instance, FIGS. 8A and 8B illustrate varying approaches of an ultrasonic tip 82 including a radially asymmetric distal region 84B for sensing and/or resecting patient tissue. FIG. 8A illustrates a manual approach of the distal region 84B relative to patient tissue without the guidance of a robotic manipulator 16. In this example, the vector $\underline{u}$ normal to the cutting teeth of the distal region 84 is not aligned with the vector $\underline{v}$ normal to the tissue to be sensed or resected, which may result in a relatively less optimal resection rate and potentially erroneous tissue sensing. Conversely, FIG. 8B illustrates an approach of the distal region 84B relative to patient tissue with the guiding support of the robotic manipulator 16, in which the vector $\underline{u}$ is aligned with the vector $\underline{v}$, resulting in relatively more optimal tissue resection and/or more accurate sensing.

Referring again to FIG. 6, the control system, such as the navigation controller 26, may thus be configured to obtain one or more target parameters corresponding to the specific tip 82 currently coupled to the handpiece 86 so as to provide improved sensing and/or resection with the tip 82, such as from the target data 56. For instance, in some implementations, the control system, such as the ultrasonic controller 150, may be configured to identify the type of the ultrasonic tip 82 based on data read from the tip memory 190 when the ultrasonic instrument 18 is coupled to the control console 80. The ultrasonic controller 150 may forward the tip identification information to the navigation controller 26, which in turn may be configured to retrieve the target parameters corresponding to the identified tip from the target data 56. As an alternative example, one or more target parameters specific to the tip 82 may be stored on the tip memory 190, which may be read by the ultrasonic controller 150 as described above and forwarded to the navigation controller 26 for implementation.

For example and without limitation, the target parameters for a given tip 82 may include one or more of a target sense force to apply to the ultrasonic instrument 18 for sensing a type of patient tissue being contacted by the distal region 84 of the tip 82, a target sense pose for the distal region 84 of tip 82 relative to contacted tissue for sensing a type of the contacted patient tissue, a target resection force to apply to the ultrasonic instrument 18 for resecting patient tissue with the tip 82, and a target resection pose for the distal region 84 of the tip 82 relative to contacted patient tissue for resecting the contacted patient tissue. Thereafter, as the ultrasonic instrument 18 is maneuvered to sense and/or resect patient tissue as described above, the control system, such as the robotic controller 64 at the direction of the navigation controller 26, may be configured to control the robotic manipulator 16 to maneuver and/or guide movement of the ultrasonic instrument 18 in accordance with the relevant target parameter(s). For instance, the control system, such as robotic controller 64 at the direction of the navigation controller 26, may be configured to maneuver and/or guide movement the ultrasonic instrument 18 to a target pose based on the tracking data generated by the ultrasonic controller 150, and may be configured to control the robotic manipulator 16 to regulate the force being applied on the ultrasonic instrument 18 toward contacted patient tissue to a target force based on the force data generated by the force-torque sensor(s) 66 or inferred from electrical characteristics of the ultrasonic instrument 18 as described above.

In some implementations, the control system, or more particularly the navigation controller 26 and/or ultrasonic controller 150, may be configured to determine whether patient tissue or another object is contacting a portion of the ultrasonic instrument 18 proximal the distal region of the tip 82, which may place an undesired load on the tip 82. Such undesired load condition may result in an inaccurate tissue type determination, such as when the ultrasonic instrument 18 is operating in the sense mode, and/or relatively less optimal resection rates, such as when the ultrasonic instrument 18 is operating in the resection mode. For instance, the control system, such as the navigation controller 26, may be configured to determine an undesired load condition responsive to the tracking data indicating a tracked object is within a set distance of the ultrasonic instrument 18 in the known coordinate system.

Additionally or alternatively, the control system, such as the navigation controller 26, may be configured to determine an undesired load condition responsive to an electrical characteristic of the ultrasonic instrument 18, such as the voltage $v_s$ of the sourced to the AC drive signal or the mechanical impedance $Z_M$ of the ultrasonic instrument 18, being greater than a corresponding threshold. In some examples, the threshold may be dynamically determined based on the tracked position of the distal region 84 of the tip 82 relative to the tissue-density map in the known coordinate system. For instance, the navigation controller 26 may be configured to determine an expected electrical characteristic of the ultrasonic instrument 18 based on the tissue density indicated by the tissue-density map at the position of the distal region 84 of the tip 82 indicated by the tracking data, and determining the threshold by adding an offset value to the expected electrical characteristic. Should the determined electrical characteristic of the ultrasonic instrument 18 be greater than this threshold, the ultrasonic controller 26 may be configured to assume that an undesired load condition is present.

Responsive to determining that an undesired load condition exists, the control system, such as the navigation controller 26 or the ultrasonic controller 150, may be configured, such as via one of the user interfaces 30A, 30B, to instruct the user to change position of the ultrasonic instrument 18. Additionally or alternatively, responsive to determining that an undesired load condition exists, the control system, such as the robotic controller 64 at the direction of the navigation controller 26, may be configured to maneuver and/or guide movement of the ultrasonic instrument 18 to a position in which no such undesired load condition exits, such as based on the tracking data and/or the virtual objects associated with the tracked objects as described above.

As previously described, the robotic manipulator 16 may be operable in various modes, such as an autonomous mode, a hands-on mode, and a telemanipulation mode. In each mode, the control system may control the robotic manipulator 16 as a function of the tracking data generated by the surgical navigation system 14. The tracking data may indicate a pose of the ultrasonic instrument 18 relative to tracked objects of interest in the surgical workspace, such as varying patient tissues and instruments located in or adjacent the target site, and correspondingly of virtual objects associated with the tracked objects of interest, in a known coordinate system. As previously described, the control system, such as the navigation controller 26, may be configured to reference previously determined relationship data 50 indicating registrations between the tracked objects of interest and trackers 20 associated with the objects and localized by the surgical navigation system 14 in the known coordinate system so as to generate the tracking data. In some cases, the relationship data 50 and/or virtual objects may need to be recalibrated intraoperatively, such as due to tissue shift during a surgical procedure. The following method may be implemented by the control system, such as at least the navigation controller 26, to track resection status of a surgical procedure, and recalibrate the relationship data 50 and/or virtual objects as needed.

To begin, the control system, such as the navigation controller 26, may receive a medical image of a target site and non-targeted objects adjacent the target site. This image can be acquired through various imaging techniques such as MRI scans, radiological scans, or computed tomography (CT) scans. The image may provide a detailed view of the patient's anatomy, including the specific tissue targeted for resection, the surrounding tissue that is not targeted (e.g., tissue to be avoided), and hardware desired to be avoided (e.g., metal screws and rods), such as in the case of a revision surgery.

The control system, such as the navigation controller 26, may be configured to generate one or more virtual objects associated with the objects in the medical image in the known coordinate system. For instance, the control system, such as the navigation controller 26, may be configured to apply a segmentation algorithm to the medical image to identify boundaries for various objects in the image, such as various tissue types, and/or identify such boundaries based on user interaction with the medical image via the user interface 30A. The control system, such as the navigation controller 26, may then be configured to apply the relationship data 50 to the medical image to transform the identified boundaries from a coordinate system of the medical image to the known coordinate system as described above, and generate virtual objects associated with the boundaries in the known coordinate system for controlling the robotic manipulator 16. The control system, such as the navigation controller 26, may then be configured to continuously track the pose of the ultrasonic instrument 18 relative to the virtual objects in the known coordinate system based on the tracking data described above. This real-time tracking may allow the control system, such as the robotic controller 64 as directed by the navigation controller 26, to control the robotic manipulator 16 to maneuver and/or guide movement of the ultrasonic instrument 18 relatively precisely according to the surgical plan 54.

When the robotic manipulator 16 is operating in the telecommunication or hands-on mode, the control system, such as the robotic controller 64 at the direction of the navigation controller 26, may be configured to control to the robotic manipulator 16 to provide haptic feedback felt by the surgeon that promotes optimal positioning and handling of the ultrasonic instrument 18 to resect the target tissue while avoiding the non-targeted objects. To this end, and as illustrated in the exemplary screen 216 of FIG. 10A that may be generated and displayed by the control system, such as the navigation controller 26, the control system, such the navigation controller 26, may be configured to generate a virtual map 218 in the known coordinate system based on the medical image. The virtual map 218 may define a plurality of regions, each being characterized and associated with a displayed label. Each region may correspond to a voxel of the medical image. For instance, at least initially, the virtual map 218 may include one or more target regions 220 of the known coordinate system that correspond to the target site according to the medical image. Each target region 220 may be associated with a "non-visited" label on a display of the user interface 30A, such as via a corresponding key or responsive to a user hovering a pointer over the target region 220. In some implementations, the virtual map 218 may also define one or more non-target regions 222 of the known coordinate system that correspond to the non-targeted objects according to the medical image, which may each be associated with a "to be avoided" label on a display of user interface 30A.

Each region of the virtual map 218 may be represented in the known coordinate system by a corresponding haptic virtual object, which may include a control property set to either attract or resist movement of the distal region 84 of the tip 82 relative to the associated region. For instance, the control property for each target region 220 may be set to attract the distal region 84 of the tip 82, and the control property for each non-targeted region 222 may be set to resist movement of the distal region 84 of the tip 82 towards the region, and/or to prevent the distal region 84 of the tip 82 from entering the non-target region 222. The virtual map 218 may thus define a potential field that guides motion of the ultrasonic instrument 18 relative to the target site and non-targeted objects adjacent target site. In some implementations, each region 220, 222 of the virtual map 218 may also be associated with tissue data indicating a type of tissue in the region 220, 222, such as according to the medical image and/or based on user input.

The control system, such as the ultrasonic controller 150, may then be configured to determine whether to activate the ultrasonic instrument 18, such as based on a corresponding signal from the foot pedal 138. If so, then the control system, such as the ultrasonic controller 150, may be configured to generate and source an AC drive signal to the ultrasonic instrument 18 that induces vibrations in the tip 82 of the ultrasonic instrument 18. In some implementations, at least upon initial activation of the ultrasonic instrument 18, the sourced AC drive signal may be configured to operate the ultrasonic instrument 18 in the sense mode described above. Around this time, the control system, such as the navigation controller 26, may also be configured to begin tracking a position of a distal region 84 of the tip 82 relative to the regions 220, 222 in the known coordinate system, and the control system, such as the robotic controller 64 at the direction of the navigation controller 26, may be configured to control the robotic manipulator 16 to maneuver and/or guide movement of the distal region 84 of the tip 82 to one of the targeted regions 220 while avoiding the non-target regions 222 according to the tracking data and the virtual map (e.g., according to the control properties of the haptic virtual objects defined by the virtual map).

Responsive to the tracking data indicating that the distal region 84 of the tip 82 is positioned at one of the target regions 220, the control system, such as the ultrasonic controller 150, may be configured to obtain at least one electrical characteristic of the AC drive signal. For instance, the control system may be configured to determine a frequency $f$, a voltage $v_s$, and/or a current $i_s$ of the AC drive signal. The control system, such as the ultrasonic controller 150, may then be configured to determine whether the electrical characteristic(s) correspond to contact with tissue targeted for resection, or more specifically, whether the electrical characteristic(s) corresponds to contact with the tissue type associated with the target region 220, such as by referencing the tissue type data 172 as described above. If so, then the control system, such as the ultrasonic controller 150 in cooperation with the navigation controller 26, may be configured to transition operation of the ultrasonic instrument 18 to the resection mode to resect the targeted tissue from the target region 220 as described above. Contemporaneously, the control system, such as the navigation controller 26, may be configured to change for label for the target region 220 from "non-visited" to "in progress."

Following such resection, the control system, such as the navigation controller 26, may be configured to recharacterize the target region 220 as a resected region 224 (FIG. 10B), and likewise change the displayed label associated with the recharacterized resected region 224 to "resected." The control system, such as the navigation controller 26, may also be configured to reset the control property associated with the resected region 224 to exhibit no attractive force relative to the distal region 84 of the tip 82. Alternatively, such as if the virtual map 218 does not indicate any remaining target regions 220 adjacent the resected region 224, the control system, such as the navigation controller 26, may be configured to reset the control property associated with the resected region 224 to resist movement of the distal region 84 of the tip 82 towards the resected region 224 and/or prevent movement of the distal region 84 of the tip 82 into the resected region 224.

Conversely, responsive to determining that the at least one electrical characteristic of the AC drive signal determined when the tracking data indicated contact with a given target region 220 does not correspond to contact with targeted tissue or to the tissue data associated with the target region 220, the control system, such as the navigation controller 26, may be configured to recharacterize the target region 220 as a mismatched region 226 (FIG. 10B), and likewise change the displayed label associated with the recharacterized mismatched region 226 to "mismatched. The control system, such as the navigation controller 26, may also be configured to reset the control property associated with the mismatched region 226 to resist movement of the distal region of the tip 82 towards the mismatched region 226 and/or prevent movement of the distal region 84 of the tip 82 into the mismatched region 226. In such case, the control system, such as the ultrasonic controller 150 at the direction of the navigation controller 26, may also be configured to either continue or transition operation of the ultrasonic instrument 18 into the sense mode, such as until further targeted tissue is detected in another target region 220 or until the virtual map 218 is recalibrated via an updated medical image or based on determined electrical characteristic(s) as described below.

As the ultrasonic instrument 18 is maneuvered relative to the surgical site, the control system, such as the navigation controller 26, may be configured to display the resection status of the procedure. For instance, as illustrated in FIG. 10B, the control system may generate and display a screen 228, such as on the user interface 30A, that illustrates each resected region 224, each remaining target region 220, and any mismatched regions 226.

Periodically and/or responsive to characterizing a given target region 220 as mismatched, the control system, such as the navigation controller 26, may be configured to determine whether to recalibrate the virtual map, such as based on a further medical image similar to as described above, or based on the electrical characteristic(s) determined for the previous visited regions (e.g., resected regions 224, mismatched regions 226). For instance, the control system, such as the navigation controller 26, may be configured to determine whether a predefined number or volume of regions surrounding each mismatched region has been visited and characterized, and if so, whether the electrical characteristic(s) determined for the visited regions generally represent a consistent boundary between targeted tissue and non-targeted objects. If so, then the control system, such as the navigation controller 26, may be configured to characterize the mismatched region(s) 226 adjacent the represented boundary as either a target region 220 or non-target region 222 depending on which side of the boundary the mismatched region 226 is located, and also change the label associated with each recharacterized region back to "non-visited" or "to be avoided" as appropriate. The control system, such as the navigation controller 26, may also be configured to reset the control property associated with each recharacterized target region 220 to attractive and associated with each recharacterized non-target region 222 to resistive as described above.

As the target regions 220 are visited and characterized, the control system, such as the navigation controller 26, may also be configured to repeatedly determine whether any remaining target regions 220 are unreachable, and if so, change the displayed label associated with such target region 220 to "not reachable." For instance, an unvisited target region 220 may be determined as unreachable if there is no path to the target region 220 that avoids non-target regions 222 and/or mismatched regions 226. Similarly, the control system, such as the navigation controller 26, may also be configured to repeatedly determine whether any unvisited target regions 220 are reachable. If not, then the control system, such as the navigation controller 26 in cooperation with at least one of the ultrasonic controller 150 and the robotic controller 64, may be configured to indicate such condition via the user interface 30A, to cease driving the ultrasonic instrument 18, and/or control the robotic manipulator 16 to remove the ultrasonic instrument 18 from the surgical site.

In some implementations, as the ultrasonic instrument 18 is maneuvered relative the surgical site, the tracking data may indicate that the distal region 84 of the tip 82 has returned to a resected region 224. When such condition is present, the control system, such as the ultrasonic controller 150 in cooperation with the navigation controller 26, may be configured to again obtain at least one electrical characteristic of the ultrasonic instrument 18, and determine whether the electrical characteristic(s) indicates targeted tissue continues to be disposed in the resected region 224. If so, in addition to again controlling the robotic manipulator 16 and/or ultrasonic instrument 18 to resect the tissue in the resected region 224, to the extent other resected regions 224 are adjacent the present resected region 224, the control system, such as the navigation controller 26, may be configured to recharacterize such resected regions 224 as target regions 220, adjust the displayed label of these recharacterized target regions 220 to "non-visited," and also reset the control property associated these recharacterized target regions 220 to attractive so as to influence the distal region 84 to again visit and resect any remaining tissue in the recharacterized target regions 220.

The present disclosure provides several benefits in the field of surgical procedures, particularly in tissue resection. Specifically, the use of a robotic manipulator to maneuver and/or guide movement of an ultrasonic instrument based on feedback from the ultrasonic instrument improves the precision of tissue resection. The system can determine contact with a volume of patient tissue targeted for resection based on electrical characteristics of the AC drive signal and/or a force being applied to the ultrasonic instrument, reducing the potential of damaging healthy tissue. Moreover, the system improves tissue resection and sensing with the ultrasonic instrument by precisely controlling the level of force applied to the ultrasonic instrument, the pose of the ultrasonic instrument relative to the patient tissue, and/or applying a predefined movement pattern to the ultrasonic instrument during resection that is specific to the tip of the ultrasonic instrument, resulting in more precise cuts and implant placement, and reducing the risk of skid and sideslip. The system can make real-time adjustments during the surgical procedure, such as updating a pose of a virtual object associated with the target volume in a known coordinate system based on the obtained electrical characteristics of the AC drive signal driving the ultrasonic instrument, allowing for immediate response to changes in the surgical field. As a result, the risk of incomplete resection is reduced. Any one of these features reduces the learning curve for surgeons by automating complex tasks such as determining contact with the volume of patient tissue targeted for resection and controlling the force applied during resection, potentially resulting in improved surgical outcomes for the patient.

The foregoing description is merely illustrative in nature and is in no way intended to limit the disclosure, its application, or uses. The broad teachings of the disclosure can be implemented in a variety of forms. Therefore, while this disclosure includes particular examples, the true scope of the disclosure should not be so limited since other modifications will become apparent upon a study of the drawings, the specification, and the following claims. It should be understood that one or more steps within a method may be executed in different order (or concurrently) without altering the principles of the present disclosure. Further, although each of the examples is described above as having certain features, any one or more of those features described with respect to any example of the disclosure can be implemented in and/or combined with features of any of the other examples, even if that combination is not explicitly described. In other words, the described examples are not mutually exclusive, and permutations of one or more examples with one another remain within the scope of this disclosure.

In the examples and implementations described herein, the term "pose" may be replaced with "position and/or orientation", and the term "poses" may be replaced with "positions and/or orientations", without departing from the scope of this disclosure. Similarly, the terms "position" and "orientation" may be replaced with "pose" without departing from the scope of this disclosure.

Although the surgical system 10 is described as having multiple controllers for implementing various functions, features, and processes, it is within the scope of this disclosure for two or more of such controllers to be integrated in a single controller configured to provide the described functions, features, and processes of the separate controllers. The functions, features, and processes of a given controller described herein may also be distributed across a plurality of controllers. In this application, it will be understood that one or more controllers being configured to implement a plurality of functions, features, and/or processes may include a plurality of controllers each configured to implement a different one of the functions, features, and/or processes, and that one or more controllers being configured to implement a given function, feature, or process may include a plurality of controllers that are configured to cooperate, such as via communications therebetween, to implement the given function, feature, or process.

Spatial and functional relationships between elements (for example, between controllers, circuit elements, semiconductor layers, etc.) are described using various terms, including "connected," "engaged," "coupled," "adjacent," "next to," "on top of," "above," "below," and "disposed." Unless explicitly described as being "direct," when a relationship between first and second elements is described in the above disclosure, that relationship can be a direct relationship where no other intervening elements are present between the first and second elements, but can also be an indirect relationship where one or more intervening elements are present (either spatially or functionally) between the first and second elements.

As used herein, the phrase at least one of A, B, and C should be construed to mean a logical (A OR B OR C), using a non-exclusive logical OR, and should not be construed to mean "at least one of A, at least one of B, and at least one of C." The term subset does not necessarily require a proper subset. In other words, a first subset of a first set may be coextensive with (equal to) the first set.

In the FIGS., the direction of an arrow, as indicated by the arrowhead, generally demonstrates the flow of information (such as data or instructions) that is of interest to the illustration. For example, when element A and element B exchange a variety of information, but information transmitted from element A to element B is relevant to the illustration, the arrow may point from element A to element B. This unidirectional arrow does not imply that no other information is transmitted from element B to element A. Further, for information sent from element A to element B, element B may send requests for, or receipt acknowledgements of, the information to element A.

In this application, including the definitions below, the term "controller" or "module" may be replaced with the term "circuit." The term "controller" may refer to, be part of, or include: at least one Application Specific Integrated Circuit (ASIC); at least one programmable system on a chip (PSoC); at least one digital, analog, or mixed analog/digital discrete circuit; at least one digital, analog, or mixed analog/digital integrated circuit; at least one combinational logic circuit; at least one field programmable gate array (FPGA); at least one processor (shared, dedicated, or group) that executes code; at least one memory circuit (shared, dedicated, or group) that stores code executed by the at least one processor; other suitable hardware components that provide the described functionality; or a combination of some or all of the above, such as in a system-on-chip.

The controller may include one or more interface circuits with one or more transceivers, such as radio frequency (RF) or optical based transceivers (e.g., infrared (IR)). In some examples, the interface circuit(s) may implement wired or wireless interfaces that connect to a local area network (LAN) or a wireless personal area network (WPAN). Examples of a LAN are Institute of Electrical and Electronics Engineers (IEEE) Standard 802.11-2016 (also known as the WIFI wireless networking standard) and IEEE Standard 802.3-2015 (also known as the ETHERNET wired networking standard). Examples of a WPAN are the BLUETOOTH wireless networking standard from the Bluetooth Special Interest Group and IEEE Standard 802.15.4.

The controller may communicate with other controllers using the interface circuit(s). Although the controller may be depicted in the present disclosure as logically communicating directly with other controllers, in various implementations the controller may actually communicate via a communications system. The communications system may include physical and/or virtual networking equipment such as hubs, switches, routers, gateways and transceivers. In some implementations, the communications system connects to or traverses a wide area network (WAN) such as the Internet. For example, the communications system may include multiple LANs connected to each other over the Internet or point-to-point leased lines using technologies including Multiprotocol Label Switching (MPLS) and virtual private networks (VPNs).

In various implementations, the functionality of the controller may be distributed among multiple controllers that are connected via the communications system. For example, multiple controllers may implement the same functionality distributed by a load balancing system. In a further example, the functionality of the controller may be split between a server (also known as remote, or cloud) controller and a client (or, user) controller.

Some or all hardware features of a controller may be defined using a language for hardware description, such as IEEE Standard 1364-2005 (commonly called "Verilog") and IEEE Standard 1076-2008 (commonly called "VHDL"). The hardware description language may be used to manufacture and/or program a hardware circuit. In some implementations, some or all features of a controller may be defined by a language, such as IEEE 1666-2005 (commonly called "SystemC"), that encompasses both code, as described below, and hardware description.

The term code, as used above, may include software, firmware, and/or microcode, and may refer to programs, routines, functions, classes, data structures, and/or objects. The term shared processor circuit encompasses a single processor circuit that executes some or all code from multiple controllers. The term group processor circuit encompasses a processor circuit that, in combination with additional processor circuits, executes some or all code from one or more controllers. References to multiple processor circuits encompass multiple processor circuits on discrete dies, multiple processor circuits on a single die, multiple cores of a single processor circuit, multiple threads of a single processor circuit, or a combination of the above. The term shared memory circuit encompasses a single memory circuit that stores some or all code from multiple controllers. The term group memory circuit encompasses a memory circuit that, in combination with additional memories, stores some or all code from one or more controllers.

The term memory circuit is a subset of the term computer-readable medium. The term computer-readable medium, as used herein, does not encompass transitory electrical or electromagnetic signals propagating through a medium (such as on a carrier wave); the term computer-readable medium may therefore be considered tangible and non-transitory. Non-limiting examples of a non-transitory computer-readable medium are nonvolatile memory circuits (such as a flash memory circuit, an erasable programmable read-only memory circuit, or a mask read-only memory circuit), volatile memory circuits (such as a static random access memory circuit or a dynamic random access memory circuit), magnetic storage media (such as an analog or digital magnetic tape or a hard disk drive), and optical storage media (such as a CD, a DVD, or a Blu-ray Disc).

The apparatuses and methods described in this application may be partially or fully implemented by a special purpose computer created by configuring a general purpose computer to execute one or more particular functions embodied in computer programs. The functional blocks and flowchart elements described above may serve as software specifications, which can be translated into the computer programs by the routine work of a skilled technician or programmer.

The computer programs may include processor-executable instructions that are stored on at least one non-transitory computer-readable medium. The computer programs may also include or rely on stored data. The computer programs may encompass a basic input/output system (BIOS) that interacts with hardware of the special purpose computer, device drivers that interact with particular devices of the special purpose computer, one or more operating systems, user applications, background services, background applications, etc.

The computer programs may include: (i) descriptive text to be parsed, such as HTML (hypertext markup language), XML (extensible markup language), or JSON (JavaScript Object Notation), (ii) assembly code, (iii) object code generated from source code by a compiler, (iv) source code for execution by an interpreter, (v) source code for compilation and execution by a just-in-time compiler, etc. As examples only, source code may be written using syntax from languages including C, C++, C#, Objective C, Swift, Haskell, Go, SQL, R, Lisp, Java®, Fortran, Perl, Pascal, Curl, OCaml, JavaScript®, HTML5 (Hypertext Markup Language 5th revision), Ada, ASP (Active Server Pages), PHP (PHP: Hypertext Preprocessor), Scala, Eiffel, Smalltalk, Erlang, Ruby, Flash®, Visual Basic®, Lua, MATLAB, SIMULINK, LabVIEW, and Python®.

**Claims**

1. A robotic surgical system (10) comprising:

    an ultrasonic instrument (18) comprising an irrigation pathway, a tip (82), and an ultrasonic transducer (90) coupled to the tip (82), the ultrasonic transducer (90) configured to vibrate the tip (82) responsive to receiving an AC drive signal;
    a power supply (154) coupled to the ultrasonic instrument (18) and configured to generate the AC drive signal applied to the ultrasonic transducer (90) of the ultrasonic instrument (18);
    a robotic manipulator (16) coupled to the ultrasonic instrument (18) and configured to control movement of the ultrasonic instrument (18) to resect a target volume of patient tissue; and
    a control system (26, 64, 150) coupled the power supply (154) and the robotic manipulator (16) and configured to:

        operate the power supply (154) to source the AC drive signal to the ultrasonic instrument (18) to induce vibrations in the tip (82) of the ultrasonic instrument (18);
        obtain a first characteristic of the sourced AC drive signal;
        determine whether a distal region (84) of the tip (82) is vibrating against a portion of the target volume based on the first characteristic; and
        operate the robotic manipulator (16) to control movement of the ultrasonic instrument (18) relative to the patient tissue based on the determination of whether the distal region (84) of the tip (82) is vibrating against a portion of the target volume.

2. The robotic surgical system (10) of claim 1, wherein the first characteristic is a frequency or a voltage of the sourced AC drive signal.

3. The robotic surgical system (10) of claim 1, wherein the control system (26, 64, 150) is configured to:

    determine a measure of force being applied to the ultrasonic instrument (18) when the first characteristic is obtained; and
    determine whether the distal region (84) of the tip (82) is vibrating against the target volume portion based on the first characteristic and the force measure.

4. The robotic surgical system (10) of claim 3, comprising a navigation system (14) configured to generate tracking data indicative of a position of the distal region (84) of the tip (82) relative to the patient tissue, and the control system (26, 64, 150) is configured to:

    receive a medical image of the patient tissue;
    generate a tissue-density map for the patient tissue based on the medical image;
    obtain a second characteristic of the AC drive signal when the tracking data indicates the distal region (84) of the tip (82) is vibrating against the target volume portion; and
    determine the force measure based on a portion of the tissue-density map corresponding to the target volume portion and the second characteristic.

5. The robotic surgical system (10) of any one of claims 1-4, wherein the control system (26, 64, 150) is configured to,

responsive to determining that the distal region (84) of the tip (82) is vibrating against a portion of the target volume:

determine a target resection force to apply to the ultrasonic instrument (18) for resection of the target volume portion based on the first characteristic;

operate the ultrasonic instrument (18) to resect the target volume portion; and

operate the robotic manipulator (16) to regulate a force applied to the ultrasonic instrument (18) during the resection the target volume portion based on the target resection force.

6. The robotic surgical system (10) of any one of claims 1-5, wherein the control system (26, 64, 150) is configured to:

responsive to determining that the distal region (84) of the tip (82) is vibrating against a portion of the target volume, operate the ultrasonic instrument (18) to resect the target volume portion;

select a predefined motion to apply to the ultrasonic instrument (18) that simulates a tactile feel corresponding to resection of the target volume portion based on the first characteristic; and

operate the robotic manipulator (16) during the resection of the target volume portion to apply the predefined motion to the ultrasonic instrument (18).

7. The robotic surgical system (10) of any one of claims 1-6, comprising a navigation system (14) configured to generate tracking data indicative of a pose of the distal region (84) of the tip (82) relative to the patient tissue, wherein the tip (82) is releasably coupled to a handpiece (86) of the ultrasonic instrument (18), the distal region (84) of the tip (82) is radially asymmetric, and the control system (26, 64, 150) is configured to:

identify a type of the tip (82) coupled to the handpiece (86);

determine a target sense pose for the distal region (84) of the tip (82) relative to the patient tissue for sensing whether the distal region (84) of the tip (82) is vibrating against the target volume portion based on the identified tip (82) type;

operate the robotic manipulator (16) to control movement of the ultrasonic instrument (18) such that the distal region (84) of the tip (82) is in the target sense pose relative to the patient tissue based on the tracking data; and

obtain the first characteristic when the distal region (84) of the tip (82) is in the target sense pose relative to the patient tissue.

8. The robotic surgical system (10) of any one of claims 1-7, comprising a navigation system (14) configured to generate tracking data indicative of a position of the distal region (84) of the tip (82) relative to the target volume, wherein the tip (82) is releasably coupled to a handpiece (86) of the ultrasonic instrument (18), the distal region (84) of the tip (82) is radially asymmetric, and the control system (26, 64, 150) is configured to:

identify a type of the tip (82) coupled to the handpiece (86);

determine a target resection pose for the distal region (84) of the tip (82) relative to the target volume for resecting the target volume based on the identified tip (82) type;

responsive to determining that the distal region (84) of the tip (82) is vibrating against a portion of the target volume based on the first characteristic, operate the ultrasonic instrument (18) to resect the target volume portion; and

operate the robotic manipulator (16) to control movement of the ultrasonic instrument (18) such that, during operation of the ultrasonic instrument (18) to resect the target volume portion, the distal region (84) of the tip (82) is in the target resection pose relative to the target volume portion based on the tracking data.

9. The robotic surgical system (10) of any one of claims 1-8, wherein the tip (82) is releasably coupled to a handpiece (86) of the ultrasonic instrument (18), and the control system (26, 64, 150) is configured to:

determine a target sense force specific to the tip (82) coupled to the handpiece (86) to apply to the ultrasonic instrument (18) for sensing whether the distal region (84) of the tip (82) is vibrating against a portion of the target volume;

operate the robotic manipulator (16) to regulate a force applied to the ultrasonic instrument (18) based on the target sense force; and

obtain the first characteristic when the robotic manipulator (16) is being operated to regulate the force applied to the ultrasonic instrument (18) based on the target sense force.

10. The robotic surgical system (10) of claim 9, wherein the control system (26, 64, 150) is configured to:

operate the robotic manipulator (16) to control movement of the ultrasonic instrument (18) to reposition the distal region (84) of the tip (82) relative to the patient tissue after obtaining the first characteristic and to regulate the force applied to the ultrasonic instrument (18) after the distal region (84) of the tip (82) is repositioned based on the target sense force;

obtain a third characteristic of the AC drive signal when the robotic manipulator (16) is being operated to regulate the force applied to the ultrasonic instrument (18) based on the target sense force after the distal region (84) of the tip (82) is repositioned;

determine whether the distal region (84) of the tip (82) is vibrating against a second portion of the target volume based on the third characteristic; and

operate the robotic manipulator (16) to control movement of the ultrasonic instrument (18) relative to the patient tissue based on the determination of whether the distal region (84) of the tip (82) is vibrating against the second portion of the target volume.

11. The robotic surgical system (10) of any one of claims 1-10, wherein the tip (82) is releasably coupled to a handpiece (86) of the ultrasonic instrument (18), and the control system (26, 64, 150) is configured to:

identify a type of the tip (82) coupled to the handpiece (86);

select a predefined movement pattern for the distal region (84) of the tip (82) during resection of the target volume based on the identified tip (82) type;

responsive to determining that the distal region (84) of the tip (82) is vibrating against a portion of the target volume based on the first characteristic, operate the ultrasonic instrument (18) to resect the target volume portion; and

operate the robotic manipulator (16) to move the distal region (84) of the tip (82) in the predefined movement pattern during the resection of the target volume portion.

12. A method of operating the robotic surgical system (10) of any preceding claim.

13. A method for operating a robotic manipulator (16) coupled to an ultrasonic instrument (18) to control movement of the ultrasonic instrument (18) to resect a target volume of patient tissue, the method comprising:

driving the ultrasonic instrument (18) with an AC drive signal to induce vibrations in a tip (82) of the ultrasonic instrument (18);

positioning the ultrasonic instrument (18) so that a distal region (84) of the tip (82) is vibrating against a portion of the patient tissue;

obtaining a first characteristic of the AC drive signal when the distal region (84) of the tip (82) is vibrating against the tissue portion;

determining whether the tissue portion corresponds to the target volume based on the first characteristic; and

operating the robotic manipulator (16) to control movement of the ultrasonic instrument (18) relative to the patient tissue based on the determination of whether the tissue portion corresponds to the target volume.

**FIG. 1**

**FIG. 2**

**FIG. 3**

EP 4 691 387 A1

82

84A

**FIG. 4A**

82

84B

**FIG. 4B**

82

84C

**FIG. 4C**

**FIG. 5A**

**FIG. 5B**

FIG. 6

FIG. 7

FIG. 8B

FIG. 8A

**FIG. 9A**

**FIG. 9B**

**FIG. 10A**

**FIG. 10B**

EP 4 691 387 A1

Europäisches
Patentamt
European
Patent Office
Office européen
des brevets

# EUROPEAN SEARCH REPORT

**Application Number**

EP 25 19 3732

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2023/196536 A1 (STRYKER CORP [US]) 12 October 2023 (2023-10-12) * figures 1-4,13-14, 18-20,23-24,26 * * paragraphs [0038] - [0039], [0161], [0183], [0231], [0254], [0259], [0298], [0318], [0336] * | 1-13 | INV. A61B17/32 A61B34/20 A61B34/30 ADD. A61B17/00 A61B90/00 |
| A | US 2021/308872 A1 (BOWLING DAVID GENE [US] ET AL) 7 October 2021 (2021-10-07) * figures 1,11,26, 28A-G * * paragraphs [0402] - [0473] * * claim 1 * | 1-13 | |
| A | WO 2021/248062 A1 (STRYKER CORP [US]) 9 December 2021 (2021-12-09) * figure 5 * | 1-13 | |
| A | US 2012/209303 A1 (FRANKHOUSER PAUL L [US] ET AL) 16 August 2012 (2012-08-16) * figures 22, 24, 33 * * paragraphs [0166], [0173] * | 1-13 | |

**TECHNICAL FIELDS SEARCHED (IPC)**

A61B

The present search report has been drawn up for all claims

| Place of search | Date of completion of the search | Examiner |
|---|---|---|
| The Hague | 27 November 2025 | van Poelgeest, A |

## ANNEX TO THE EUROPEAN SEARCH REPORT
## ON EUROPEAN PATENT APPLICATION NO.

EP 25 19 3732

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2025

| Patent document cited in search report | | Publication date | Patent family member(s) | | Publication date |
|---|---|---|---|---|---|
| WO 2023196536 | A1 | 12-10-2023 | AU | 2023250951 A1 | 24-10-2024 |
| | | | CN | 119325361 A | 17-01-2025 |
| | | | EP | 4504089 A1 | 12-02-2025 |
| | | | JP | 2025513016 A | 22-04-2025 |
| | | | WO | 2023196536 A1 | 12-10-2023 |
| US 2021308872 | A1 | 07-10-2021 | AU | 2014233900 A1 | 08-10-2015 |
| | | | AU | 2018202741 A1 | 10-05-2018 |
| | | | CA | 2906520 A1 | 25-09-2014 |
| | | | CN | 105392438 A | 09-03-2016 |
| | | | CN | 108451643 A | 28-08-2018 |
| | | | EP | 2967588 A2 | 20-01-2016 |
| | | | EP | 3756610 A1 | 30-12-2020 |
| | | | KR | 20150127129 A | 16-11-2015 |
| | | | KR | 20210062719 A | 31-05-2021 |
| | | | US | 2014039681 A1 | 06-02-2014 |
| | | | US | 2019269476 A1 | 05-09-2019 |
| | | | US | 2021308872 A1 | 07-10-2021 |
| | | | WO | 2014151550 A2 | 25-09-2014 |
| WO 2021248062 | A1 | 09-12-2021 | AU | 2021283983 A1 | 05-01-2023 |
| | | | CA | 3181229 A1 | 09-12-2021 |
| | | | CN | 115802962 A | 14-03-2023 |
| | | | EP | 4161411 A1 | 12-04-2023 |
| | | | EP | 4523638 A2 | 19-03-2025 |
| | | | JP | 2023528485 A | 04-07-2023 |
| | | | US | 2023200835 A1 | 29-06-2023 |
| | | | WO | 2021248062 A1 | 09-12-2021 |
| US 2012209303 | A1 | 16-08-2012 | AU | 2012214166 A1 | 12-09-2013 |
| | | | CA | 2827044 A1 | 16-08-2012 |
| | | | CN | 103458810 A | 18-12-2013 |
| | | | EP | 2672903 A1 | 18-12-2013 |
| | | | JP | 2014513564 A | 05-06-2014 |
| | | | US | 2012209303 A1 | 16-08-2012 |
| | | | US | 2016175543 A1 | 23-06-2016 |
| | | | US | 2016374723 A1 | 29-12-2016 |
| | | | WO | 2012109621 A1 | 16-08-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63679295
- US 7725162 B
- US 20200100849 A
- US 8702626 B
- US 8320711 B
- US 8190389 B
- US 8123722 B
- US 7720521 B
- US 20140364725 A
- US 20140200444 A
- US 20120245456 A
- US 20110060214 A
- US 20080281156 A
- US 20070208252 A
- US 2022054115 W
- US 9775681 B
- US 20220338938 A
- US 2023017790 W
- US 10398449 B
- WO 2022072903 A1
- WO 2016183084 A1
- US 10016209 B
- US 63589988